# EUROPEAN PATENT APPLICATION

(11) **EP 4 782 551 A1**
(43) Date of publication of application: **29.07.2026**
(21) Application number: 26153512.4
(22) Date of filing: 22.01.2026
(51) Int. Cl.: C12Q 1/6806

(54) **METHODS, KITS, AND SYSTEMS FOR IDENTIFICATION OF VECTOR COPY NUMBER AND INTEGRATION SITES**

(30) Priority: 22.01.2025 US 202563748170 P
(71) Applicant: National Resilience, LLC, Blue Ash, OH 45242 (US)
(72) Inventor: HANCOCK, Saege, Blue Ash, OH, 45242 (US); SALATHIA, Neeraj, Blue Ash, OH, 45242 (US)
(74) Representative: Fish & Richardson P.C.

(57) **Abstract**

Provided herein are methods, kits, and systems, for simultaneous identification and quantification of an insertion site of a vector in a genome and copy number integration by hybridizing primers to a sample of genomic DNA and amplifying a series of extension products. The extension products can be further amplified and analyzed for a junction integration site and copy number of the inserted vector.

## Description

### CROSS-REFERENCE TO RELATED APPLICATIONS

This application claims priority to U.S. Provisional Patent Application No. 63/748,170, filed on January 22, 2025, which is incorporated herein by reference in its entirety.

### SEQUENCE LISTING

This application contains a Sequence Listing that has been submitted electronically as an XML file named "60252-0009001_SL_ST26.XML." The XML file, created on January 21, 2026, is 36,400 bytes in size. The material in the XML file is hereby incorporated by reference in its entirety.

### BACKGROUND

Vector transduction strategies can assist in expressing a target transgene in a host cell. The average number of vector copies per genome is represented by the vector copy number (VCN). The genomic location and presence of an integrated vector can be represented by an integration site. Improvements in copy number identification and integration sites can be used to quantify and advance vector transduction.

### SUMMARY

The present disclosure provides improved methods, kits, and systems for simultaneously measuring and identifying integration sites and a copy number of vector insertion at single cell resolution. The methods provided herein can be used as multi-omic assays or in conjunction with sequencing protocols and transduction strategies. The methods, kits, and systems provided herein can be used to improve the accuracy, coverage, and throughput of integration site and copy number identification protocols.

The methods described herein can be used to determine vector DNA insertion sites via the identification of flanking human genomic DNA sequences with extreme sensitivity so as to be applicable to single cell contexts and therefore provide a level of accuracy and precision not possible by traditional bulk averaging of vector copy number and summed lists insertion site locations.

In one aspect, the disclosure provides methods for determining a junction integration site of a nucleic acid molecule in a genome and a copy number of integration, wherein the methods include, or consist of, (a) providing a sample of a genomic DNA molecule from a cell, wherein the genomic DNA molecule comprises an integrated nucleic acid molecule; (b) contacting a sequence of the integrated nucleic acid molecule with a first primer, wherein the first primer is configured to hybridize to the integrated nucleic acid molecule, and wherein the first primer includes a first primer handle including a sequence that does not hybridize to the integrated nucleic acid molecule; (c) extending the first primer to generate a first extension strand product including a sequence of the integrated nucleic acid molecule and a sequence of the genomic DNA molecule; (d) contacting the first extension strand product with a second primer, wherein the second primer is a random primer configured to anneal to the first extension strand product, and wherein the second primer includes a second primer handle configured to hybridize to an amplification primer; (e) extending the second primer to generate a second extension strand product; (f) amplifying the first extension strand product and the second extension strand product with a set of amplification primers to generate a junction-specific amplification product, wherein the set of amplification primers comprises index adaptors; and (g) identifying the junction integration site in the nucleic acid molecule in the genome, and identifying a copy number of integration, wherein identifying the junction of integration and identifying a copy number of integration occur concurrently, and wherein identifying the junction of integration and identifying a copy number of integration occur at a single cell resolution when applied using a single cell assay platform.

In some embodiments, the cell is a T-cell, a natural killer cell, or a precursor of a T-cell or a natural killer cell.

In certain embodiments, the first primer handle is biotinylated. In some embodiments, the first primer includes a barcode sequence or a hybridization tag, and/or the first primer can contain a unique molecular identifier (UMI). In some embodiments, the first primer includes at least six degenerate nucleotides and/or the first primer can be at least 20 nucleotides in length. In certain embodiments, the first primer binds to a 3' LTR region of the integrated nucleic acid molecule.

In certain embodiments, the methods includes the use of a plurality of first primers. In some embodiments, the plurality of first primers anneal to distinct sequences of the integrated nucleic acid molecule.

In some embodiments of the methods described herein, extending the first primer in (c) further includes heating the sample at a constant temperature for a duration of time. For example, the constant temperature can be about 50°C to about 100°C. In other embodiments, extending the first primer in (c) further includes heating the sample to a first temperature, cooling down the sample, and heating the sample to a second temperature after cooling down. For example, the first temperature and the second temperature can both be about 50°C to about 100°C. In some embodiments, the first temperature and the second temperature are the same or about the same.

In some embodiments, extending the first primer in (c) further includes contacting the sample with a thermostable enzyme and deoxynucleoside triphosphates (dNTPs). For example, the thermostable enzyme can include an enzyme with strand displacement activity. In certain embodiments, the dNTPs can include any one or more of dATP, dCTP, dGTP, dTTP, or dUTP, and in some examples, the dNTPs can be or include unnatural dNTPs or the dNTPs can be or include natural and unnatural dNTPs.

In some embodiments, the first extension strand product is bound to a bead.

In certain embodiments, the second primer contains a unique molecular identifier (UMI) and/or the second primer can include a barcode sequence or a hybridization tag.

In some embodiments, the second primer has or includes a random sequence. In certain embodiments, the second primer can include at least five degenerate nucleotides and/or the second primer can have a fixed 5' region.

In certain embodiments, the second primer ends with at least five to nine random nucleotides and/or the second primer is at least 20 nucleotides in length.

In some embodiments, extending the second primer in (e) further includes heating the sample at a constant temperature for a duration of time at a temperature of about 20°C to about 80°C. In certain embodiments, extending the second primer in (e) further includes contacting the sample with a low-temperature tolerant enzyme and natural or unnatural deoxynucleoside triphosphates (dNTPs), e.g., natural or unnatural dATP, dCTP, dGTP, dTTP, or dUTP.

In some embodiments, the low-temperature enzyme is or includes an exo-Klenow polymerase.

In some embodiments, the amplifying in (f) includes performing a nucleic acid amplification, e.g., a polymerase chain reaction (PCR) or an isothermal amplification reaction.

In certain embodiments, the analyzing in (g) further includes sequencing the junction-specific amplification product using a high-throughput sequencing method.

In some embodiments, the integrated nucleic acid molecule includes a chimeric antigen receptor (CAR) sequence comprising: (i) an antigen-binding domain sequence; (ii) a costimulatory domain sequence; (iii) an intracellular signaling domain sequence, e.g., a CD3ζ domain sequence; and (iv) a 3' genomic junction.

In some embodiments, the antigen-binding domain sequence is 5' to the costimulatory domain sequence, and the costimulatory domain sequence is 5' to the CD3ζ domain sequence.

In some embodiments of these methods, the CAR includes a single co-stimulatory domain, and the costimulatory domain sequence includes at least a portion of a CD28 domain sequence or at least a portion of a 4-1BB protein domain sequence. In certain embodiments, the CAR includes two or more co-stimulatory domains, and the at least a portion of a costimulatory domain sequence comprises a 4-1BB sequence.

In some embodiments, the first primer is configured to hybridize to (i), (ii), (iii), and/or (iv), and/or wherein the second primer further includes a 3' end configured to hybridize to a 3' end of the intracellular signaling domain sequence.

In certain embodiments, the second primer further includes a 3' barcode sequence or a 3' hybridization tag.

The methods described herein can further include barcoding the junction-specific amplification product using barcoding polynucleotides including a barcode and a 3' sequence complementary to the hybridization tag or the 3' hybridization tag. In these methods, the barcode includes a unique molecular identifier (UMI). In some embodiments, the barcoding polynucleotide includes a sequencing primer hybridization site 5' to the barcode sequence. In certain embodiments, the barcoding polynucleotides are attached to a solid support, e.g., a plurality of beads.

In certain embodiments of the methods described herein, the amplifying is performed *in situ* or in an intact cell.

In some embodiments of the methods described herein, the methods can further include sorting a fixed and permeabilized cell according to polypeptide surface expression analysis and/or can further include sequencing the junction-specific amplification product.

In another aspect, the present disclosure provides methods for performing quality control on a cell including or containing a chimeric antigen receptor (CAR) sequence in its genome, the methods comprising performing any of the methods described above and herein. These methods can further include identifying a junction integration site of the CAR sequence via a sequence of a 3' genomic junction of the junction-specific amplification product; comparing the junction integration site to a genomic location oncogene; and rejecting the cell when the junction integration site overlaps with an oncogene sequence.

In some embodiments these methods can further include identifying a copy number of integration of the CAR sequence in the genome of the cell by identifying a number of a 3' genomic junction sequence that is unique; comparing the copy number of integration to a threshold and; rejecting the cell when the copy number of integration exceeds the threshold.

In another aspect, the disclosure provides kits for analyzing a cell including or having a sequence of an integrated nucleic acid molecule in its genome. These kits include: a first primer configured to hybridize to the integrated nucleic acid molecule, wherein the first primer includes a first primer handle comprising a sequence that does not hybridize to the integrated nucleic acid molecule; and a second primer including a second primer handle configured to hybridize to an amplification primer, wherein the first primer and the second primer are configured to anneal to a plurality of amplification primers.

In some embodiments of these kits, the integrated nucleic acid molecule includes a chimeric antigen receptor (CAR) sequence comprising: (i) an antigen-binding domain sequence; (ii) a costimulatory domain sequence; (iii) an intracellular signaling domain sequence; and (iv) a 3' genomic junction.

In these kits, the intracellular signaling domain sequence is a CD3ζ domain sequence.

In some embodiments of these kits, the first primer can be configured to hybridize to (i), (ii), (iii), and/or (iv) and/or wherein the first primer further includes a hybridization tag or a barcode sequence, e.g., a unique molecular identifier (UMI).

In some embodiments of the kits described herein, the first primer includes at least six degenerate nucleotides and/or wherein the second primer includes at least six to 9 degenerate nucleotides. In certain embodiments, the kits can further include a reagent for nucleic acid amplification that includes a thermostable enzyme and deoxynucleoside triphosphates (dNTPs). For example, the thermostable enzyme can be selected from the group consisting of a fragment of a *Bacillus stearothermophilus* polymerase, a exo-Klenow polymerase, a Bst 2.0 polymerase, a Bst 3.0 polymerase, a SD DNA polymerase, a phi29 DNA polymerase, a T7 exo-polymerase, and any mutants thereof.

Any of the kits described herein can further include a capture nucleic acid comprising a 3' end comprising a CD3ζ domain sequence immobilized on a solid surface, e.g., a bead. In certain embodiments, the capture nucleic acid further includes a barcode sequence 5' to the CD3ζ domain sequence, wherein the barcode sequence is a unique molecular identifier (UMI). In some embodiments, the kits described herein can further include one or more of a fixative, a permeabilization agent, or a cell lysis agent.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof. Exemplary polypeptides or proteins include gene products, naturally occurring proteins, homologs, orthologs, paralogs, fragments and other equivalents, variants, fragments, and analogues of the foregoing.

The term "vector" refers to a replicon (e.g., a cosmid, a plasmid, or a phage) to which another nucleic acid segment can be attached so as to bring about the replication of the attached segment. A vector can be capable of transferring target polynucleotides (e.g., gene sequences) to target cells. The terms "vector construct," "expression vector," "gene expression vector," "gene delivery vector," "gene transfer vector," "transfer vector," and "expression cassette" can be used interchangeably to refer to an assembly that is capable of directing the expression of a sequence or gene of interest.

The term or "vector copy number" refers to the average vector copies per genome. Copy number identification can be useful for detection of vector at the genomic level. Copy number can also refer to a genetic dose of a transgene that is present in genetically engineered cells.

The term "integration site" refers to an location of integration of a vector into a host genome. The terms "integration site," "junction integration site," or "insertion site" can be used interchangeably.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "promoter" refers to a DNA sequence recognized by the transcription machinery of the cell, or introduced synthetic machinery, that can be needed to initiate the specific transcription of a polynucleotide sequence. A promoter sequence can contain components for regulatory proteins or other factors to bind, including but not limited to transcription factors and RNA polymerase. Promoter elements can be used to further increase the frequency and/or efficiency of transcriptional initiation. These elements can be located 20, 30, 40, 50, 60, 70, 80, 90, 100, 110, 120, 130, 140, or 150 base pairs upstream of a promoter start site. Promoter elements can work separately or synergistically. In some cases, a promoter sequence can be used with an enhancer. An enhancer can be a cis-acting regulatory sequence involved in the transcriptional activation of a nucleic acid sequence. In some cases, a plasmid can contain a CMV promoter with a CMV enhancer.

A promoter can be a naturally-occurring promoter or a recombinant promoter. A recombinant enhancer refers to an enhancer that is not endogenous with a nucleic acid sequence. Recombinant promoters or enhancers can be promoters or enhancers from other genes, promoters or enhancers from different viruses, or promoters or enhancers with mutations or modifications that alter expression. A promoter and/or enhancer in the present disclosure can be used in an expression cassette to enhance gene expression in a vector, cell, formulation, organ, or organism. A promoter can be constitutive, tissue-specific, and/or inducible, to control expression levels.

An example of a promoter is the immediate early cytomegalovirus (CMV) promoter sequence. This promoter sequence is a strong constitutive promoter sequence capable of driving high levels of expression of any polynucleotide sequence operatively linked thereto. In some cases, a polynucleotide comprises a CMV promoter. In some cases, a polynucleotide can comprise a promoter selected from the group consisting of, but not limited to, a simian virus 40 (SV40) early promoter, a mouse mammary tumor virus (MMTV), a human immunodeficiency virus (HIV) long terminal repeat (LTR) promoter, a MoMuLV promoter, an avian leukemia virus promoter, an Epstein-Barr virus immediate early promoter, a Rous sarcoma virus (RSV) promoter, an actin promoter, a myosin promoter, an elongation factor-1a promoter, a hemoglobin promoter, and a creatine kinase promoter. In some cases, a polynucleotide comprises a sequence encoding a poly(A) tail. In some cases, a polynucleotide comprises a 3' UTR sequence. In some cases, a polynucleotide comprises a 5' UTR sequence. In some cases, a polynucleotide comprises multiple smaller and discrete nucleotide sequences that are typically heterologous and exhibit different and measurable function(s), a promoter sequence, a translation initiating sequence, a start codon, a polyadenylation sequence, a stop codon, and/or a linker sequence.

The term "polyA signal" refers to a polyadenylation signal sequence, for example placed 3' of a transgene, which enables host factors to add a polyadenosine (polyA) tail to the end of the nascent mRNA during transcription. The polyA tail can comprise a series of 300 adenosine ribonucleotides which protects mRNA from enzymatic degradation. The polyA tail can also assist in translation. The vectors of the present disclosure can include a polyA signal sequence such as the human beta globin or rabbit beta globin polyA signals, the simian virus 40 (SV40) early or late polyA signals, the human insulin polyA signal, or the bovine growth hormone polyA signal. In one embodiment, the polyA signal sequence is the human beta globin polyA signal.

The terms "transfected" or "transformed" or "transduced" refer to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny. The term "transformed cell" or "transfected cell" can be a transformed or transfected cell in which inserted DNA can replicate either as an autonomously replicating plasmid or as part of the host chromosome. In some cases, a transfected cell or transformed cell can express the inserted DNA or RNA transiently (e.g., for brief periods of time).

The terms "nucleic acid," "nucleic acid sequence," "nucleotide sequence," or "polynucleotide sequence," and "polynucleotide" are used interchangeably and refer to a polymeric form of nucleotides of any length, either deoxyribonucleotides or ribonucleotides, or analogs thereof. The polynucleotide can be either single-stranded or double-stranded, and if single-stranded can be the coding strand or non-coding (antisense) strand. A polynucleotide can comprise modified nucleotides, such as methylated nucleotides and nucleotide analogs. The sequence of nucleotides can be interrupted by non-nucleotide components. A polynucleotide can be further modified after polymerization, such as by conjugation with a labeling component. The nucleic acid can be a recombinant polynucleotide, or a polynucleotide of genomic, cDNA, semisynthetic, or synthetic origin which either does not occur in nature or is linked to another polynucleotide in a non-natural arrangement. A single-stranded nucleic acid can be one nucleic acid strand of a denatured double-stranded DNA. Alternatively, it can be a single-stranded nucleic acid not derived from any double-stranded DNA. In one aspect, the nucleic acid can be DNA. In another aspect, the nucleic acid can be RNA Suitable nucleic acid molecules are DNA, including genomic DNA or cDNA. Other suitable nucleic acid molecules are RNA, including siRNA, shRNA, and antisense oligonucleotides.

The transgenes described include nucleic acid sequences that have been removed from their naturally occurring environment, recombinant or cloned DNA isolates, and chemically synthesized analogues or analogues biologically synthesized by heterologous systems. The terms "transgene" and "gene" are also used interchangeably and both terms encompass fragments or variants thereof encoding the target protein.

If present, modifications to the nucleotide structure can be imparted before or after assembly of the polymer. Some non-limiting examples of analogs include: 5-bromouracil, peptide nucleic acid, xeno nucleic acid, morpholinos, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudourdine, dihydrouridine, queuosine, wyosine, PNAs, and LNAs.

Unless otherwise indicated, any nucleic acid sequences are written left to right in 5' to 3' orientation; amino acid sequences are written left to right in amino to carboxy orientation, respectively.

Calculations of homology or sequence identity between sequences (the terms are used interchangeably herein) are performed as follows. To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4. The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to a nucleic acid molecule of the disclosure. BLAST protein searches can be performed with the XBLAST program, score = 50, wordlength = 3 to obtain amino acid sequences homologous to protein molecules of the disclosure. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used.

Proteins described herein can include variants in which amino acid residues from one species are substituted for the corresponding residue in another species, either at the conserved or non-conserved positions. Variants of protein molecules disclosed herein can be produced and used as described herein. Following the lead of computational chemistry in applying multivariate data analysis techniques to the structure/property-activity relationships [see for example, Wold, et al. Multivariate data analysis in chemistry. Chemometrics-Mathematics and Statistics in Chemistry (Ed.: B. Kowalski); D. Reidel Publishing Company, Dordrecht, Holland, 1984 (ISBN 90-277-1846-6] quantitative activity-property relationships of proteins can be derived using well-known mathematical techniques, such as statistical regression, pattern recognition and classification [see for example Norman et al. Applied Regression Analysis. Wiley-Interscience; 3rd edition (April 1998) ISBN: 0471170828; Kandel, Abraham et al. Computer-Assisted Reasoning in Cluster Analysis. Prentice Hall PTR, (Can 11, 1995), ISBN: 0133418847; Krzanowski, Wojtek. Principles of Multivariate Analysis: A User's Perspective (Oxford Statistical Science Series, No 22 (Paper)). Oxford University Press; (December 2000), ISBN: 0198507089; Witten, Ian H. et al Data Mining: Practical Machine Learning Tools and Techniques with Java Implementations. Morgan Kaufmann; (October 11, 1999), ISBN:1558605525; Denison David G. T. (Editor) et al Bayesian Methods for Nonlinear Classification and Regression (Wiley Series in Probability and Statistics). John Wiley & Sons; (July 2002), ISBN: 0471490369; Ghose, Arup K. et al. Combinatorial Library Design and Evaluation Principles, Software, Tools, and Applications in Drug Discovery. ISBN: 0-8247-0487-8]. The properties of proteins can be derived from empirical and theoretical models (for example, analysis of likely contact residues or calculated physicochemical property) of proteins sequence, functional and three-dimensional structures and these properties can be considered individually and in combination.

The polynucleotides described herein can be prepared by any means known in the art. For example, large amounts of the polynucleotides can be produced by replication in a suitable host cell. The natural or synthetic DNA fragments coding for a desired fragment will be incorporated into recombinant nucleic acid constructs, typically DNA constructs, capable of introduction into and replication in a prokaryotic or eukaryotic cell. Usually the DNA constructs will be suitable for autonomous replication in a unicellular host, such as yeast or bacteria, but can also be intended for introduction to and integration within the genome of a cultured insect, mammalian, plant or other eukaryotic cell lines.

The polynucleotides described herein can also be produced by chemical synthesis, e.g., by the phosphoramidite method or the tri-ester method, and can be performed on commercial automated oligonucleotide synthesizers. A double-stranded fragment can be obtained from the single stranded product of chemical synthesis either by synthesizing the complementary strand and annealing the strand together under appropriate conditions or by adding the complementary strand using DNA polymerase with an appropriate primer sequence.

When applied to a nucleic acid sequence, the term "isolated" in the context of the present disclosure denotes that the polynucleotide sequence has been removed from its natural genetic milieu and is thus free of other extraneous or unwanted coding sequences (but can include naturally occurring 5' and 3' untranslated regions such as promoters and terminators), and is in a form suitable for use within genetically engineered protein production systems. Such isolated molecules are those that are separated from their natural environment.

A "variant" nucleic acid sequence has substantial homology or substantial similarity to a reference nucleic acid sequence (or a fragment thereof). A nucleic acid sequence or fragment thereof is "substantially homologous" (or "substantially identical") to a reference sequence if, when optimally aligned (with appropriate nucleotide insertions or deletions) with the other nucleic acid (or its complementary strand), there is nucleotide sequence identity of at least 90%. Methods for homology determination of nucleic acid sequences are known in the art.

One of ordinary skill in the art appreciates that different species exhibit "preferential codon usage." As used herein, the term "preferential codon usage" refers to codons that are most frequently used in cells of a certain species, thus favoring one or a few representatives of the possible codons encoding each amino acid. For example, the amino acid threonine (Thr) can be encoded by ACA, ACC, ACG, or ACT, but in mammalian host cells ACC is the most commonly used codon; in other species, different codons can be preferential. Preferential codons for a particular host cell species can be introduced into the polynucleotides of the present disclosure by a variety of methods known in the art. Introduction of preferential codon sequences into recombinant DNA can, for example, enhance production of the protein by making protein translation more efficient within a particular cell type or species. Thus, according to the present disclosure, in addition to the gag-pol genes any nucleic acid sequence can be codon-optimized for expression in a host or target cell. In particular, the vector genome (or corresponding plasmid), the REV gene (or corresponding plasmid), the fusion protein (F) gene (or correspond plasmid) and/or the hemagglutinin-neuraminidase (HN) gene (or corresponding plasmid, or any combination thereof can be codon-optimized.

A "fragment" of a polynucleotide of interest can comprise a series of consecutive nucleotides from the sequence of the full-length polynucleotide. By way of example, a "fragment" of a polynucleotide of interest can comprise (or consist of) at least 30 consecutive nucleotides from the sequence of said polynucleotide (e.g., at least 35, 50, 75, 100, 150, 200, 250, 300, 350, 400, 450, 500, 550, 600, 650, 700, 750, 800 850, 900, 950 or 1000 consecutive nucleic acid residues of said polynucleotide). A fragment can include at least one antigenic determinant and/or can encode at least one antigenic epitope of the corresponding polypeptide of interest. Typically, a fragment as defined herein retains the same function as the full-length polynucleotide.

A thermal cycler platform can include, for example, the FilmArray^{®} multiplex PCR system, which extract and purifies nucleic acids from an unprocessed sample and performs nested multiplex PCR; and the RainDrop Digital PCR System, which is a droplet-based PCR platform using microfluidic chips.

The term "chimeric antigen receptor" or alternatively a "CAR" refers to a recombinant polypeptide comprising an extracellular antigen binding domain (e.g., a scFv), a transmembrane domain, and cytoplasmic, or intracellular, signaling domains. The intracellular signaling domain can comprise a functional signaling domain derived from a stimulatory molecule. Without wishing to be bound by theory, the intracellular signaling domain of a CAR is derived from the CD3 zeta chain that is normally found associated with a T-cell receptor complex. The CD3 zeta signaling domain can be fused with one or more functional signaling domains derived from at least one costimulatory molecule such as 4-1BB, CD27 and/or CD28.

The term "pharmaceutically acceptable" as used herein can mean approved by a regulatory agency of the Federal or a state government, or listed in the U.S. Pharmacopeia, European Pharmacopeia or other generally recognized pharmacopeia.

The term "cell line" or "producer cell line," as used herein, refers to cultured cells that can be passed (e.g., divided) more than once. In some cases, a cell like can be passed more than 2 times, more than 5 times, more than 10 times, more than 50 times, more than 100 times, or more than 150 times. In some cases, a cell line can be passed between 2-250 times. In some cases, a cell line can be passed up to 200 times.

As used herein, the terms "titer" and "yield" are used interchangeably to mean the amount of lentiviral (e.g., SIV) vector produced by one of the methods described herein. Titer can be the primary benchmark characterizing manufacturing efficiency, with higher titers generally indicating that more lentiviral vector is manufactured (e.g. using the same amount of reagents). Titer or yield can relate to the number of vector genomes that have integrated into the genome of a target cell (integration titer), which is a measure of "active" virus particles, i.e. the number of particles capable of transducing a cell. Transducing units (TU/mL also referred to as TTU/mL) is a biological readout of the number of host cells that get transduced under certain tissue culture/virus dilutions conditions, and is a measure of the number of "active" virus particles. The total number of (active + inactive) virus particles can also be determined using any appropriate means, such as by measuring either how much Gag is present in the test solution or how many copies of viral RNA are in the test solution. Assumptions are then made that a lentivirus particle contains either 2000 Gag molecules or 2 viral RNA molecules. Once total particle number and a transducing titer/TU have been measured, a particle:infectivity ratio calculated. Amino acids are referred to herein using the name of the amino acid, the three-letter abbreviation or the single letter abbreviation.

The term "nucleotide," as used herein, refers to a base-sugar-phosphate combination. A nucleotide can comprise a synthetic nucleotide. A nucleotide can comprise a nucleotide analog. A nucleotide can comprise a synthetic nucleotide analog. Nucleotides can be monomeric units of a nucleic acid sequence (e.g., deoxyribonucleic acid (DNA) and ribonucleic acid (RNA)). The term nucleotide includes ribonucleoside triphosphates adenosine triphosphate (ATP), uridine triphosphate (UTP), cytosine triphosphate (CTP), guanosine triphosphate (GTP) and deoxyribonucleoside triphosphates (dNTPs) such as dATP, dCTP, dITP, dUTP, dGTP, dTTP, or derivatives thereof. Such derivatives can include, for example, [αS]dATP, 7-deaza-dGTP and 7-deaza-dATP, and nucleotide derivatives that confer nuclease resistance on the nucleic acid molecule containing them. Synthetic nucleotide analogs can include locked nucleic acids (LNAs), bridged nucleic acids (BNAs), fluorinated nucleic acids (also known as fluoro-modified nucleic acids), and peptide nucleic acids (PNAs). As used herein, the term "locked nucleic acid" ("LNA"), generally refers to a nucleic acid analog wherein the ribose ring is "locked" with an extra bridge connecting the 2'-oxygen atom with the 4'-carbon atom of the nucleotide such as a methylene bridge (see e.g. WO 99/14226, which is incorporated by reference in its entirety herein). As used herein, the term "bridged nucleic acid (BNA)," generally refers to constrained or inaccessible nucleic acid molecules which have a fixed bridge structure at the 2'- or 4'-position. As used herein, "fluorinated nucleic acids" generally refer to nucleic acids which have incorporated a fluorine atom, often at the 2'- or 4'- position. As used herein, the term "peptide nucleic acid (PNA)," generally refers to a nucleotide analog wherein the backbone of the analog, for example a sugar backbone in DNA, is a pseudopeptide. A PNA backbone can comprise, for example, a sequence of repeated N-(2-amino-ethyl)-glycine units. A peptide nucleic acid analog can react as DNA would react in a given environment, and can additionally bind complementary nucleic acid sequences and various proteins. Due to the non-natural backbone, PNAs can be insensitive to endonuclease cleavage in situations where an endonuclease would cleave the equivalent DNA/RNA sequence. The term "nucleotide," as used herein, refers to dideoxyribonucleoside triphosphates (ddNTPs) and their derivatives. Illustrative examples of dideoxyribonucleoside triphosphates can include, but are not limited to, ddATP, ddCTP, ddGTP, ddITP, and ddTTP. A nucleotide can be unlabeled or detectably labeled, such as using moieties comprising optically detectable moieties (e.g., fluorophores). Detectable labels can include, for example, radioactive isotopes, fluorescent labels, chemiluminescent labels, bioluminescent labels and enzyme labels.

As used herein, the term "restriction endonuclease," "restriction enzyme," or grammatical equivalents thereof refers to an enzyme that originates in bacterial host defense and is understood to recognize a specific sequence on an incoming viral DNA and cleave the DNA either at the recognition sequence or at a distinct sequence site. One group of restriction endonucleases are identified as Type IIS. This group can recognize asymmetric DNA sequences and cleaves the DNA at a site outside the cleavage site that is at a defined distance from the recognition site. In some cases, type IIS restriction endonucleases cleave DNA between 1 and 20 nucleotides from the relevant recognition site.

As used herein, the term "restriction endonuclease recognition sequence" refers to a location on a nucleic acid molecule (e.g., DNA molecule) containing specific sequences of nucleotides, which are recognized by various restriction enzymes. These sequences can comprise from 4-8 base pairs to 12-40 base pairs in length. These sites can be palindromic sequences.

As used herein, the term "sample" refers to a substance (e.g., solid or liquid) that contains a target nucleic acid sequence to be amplified. The target nucleic acid can be DNA. The target nucleic acid molecule can be RNA.

As used herein, the term "template" refers to a portion of a target RNA or DNA of a sample that is amplified by a DNA polymerase to produce one or more amplified nucleic acid products.

As used herein, "amplified product," "amplified nucleic acid product," or "amplicon" refers to the end product resulting from a nucleic acid method, such as PCR or isothermal amplification.

As used herein, the term "polymerase" refers to an enzyme that produces a complementary replicate of a nucleic acid molecule using the nucleic acid as a template strand. DNA polymerases bind to the template strand and then move down the template strand adding nucleotides to the free hydroxyl group at the 3' end of a growing chain of nucleic acid. DNA polymerases synthesize complementary DNA molecules from DNA (e.g. DNA-dependent DNA polymerases) or RNA templates (e.g. RNA-dependent DNA polymerases or reverse transcriptases) and RNA polymerases synthesize RNA molecules from DNA templates (e.g. DNA-dependent RNA polymerases which participate in transcription). DNA polymerases generally use a short, preexisting RNA or DNA strand, called a primer, to begin chain growth. Some DNA polymerases replicate single-stranded templates, while other DNA polymerases displace the strand upstream of the site where they add bases to a chain.

As used herein, the term "strand displacing," when used in reference to a polymerase, refers to an activity that removes a complementary strand from base-pairing with a template strand being read by the polymerase. Example polymerases having strand displacing activity include the large fragment of *Bacillus stearothermophilus* polymerase, exo-Klenow polymerase, Bst 2.0 polymerase, Bst 3.0 polymerase, SD DNA polymerase, phi29 DNA polymerase, and sequencing-grade T7 exo-polymerase.

As used herein, "primer" or "primer sequence" refers to a linear oligonucleotide that is complementary to and anneals to a target sequence. The lower limit on primer length is determined by ability to hybridize since very short primers (e.g., less than 5 nucleotides) do not form thermodynamically stable duplexes under most hybridization conditions. Primers typically vary in length from 4 to 50 nucleotides. In some embodiments, the primer is between about 10 and 20 nucleotides in length. In some embodiments, the primer can be more than about 100 nucleotides in length. In some embodiments, the primer can be an oligonucleotide that can hybridize with a target nucleic acid sequence. In some embodiments, the primer can be described as a probe.

As used herein, the terms "amplify," "amplifies," "amplified," and "amplification" refer to any method for replicating a nucleic acid. The replication can be conducted with the use of a primer-dependent polymerase. The replication can be enzyme-free amplification. In some cases, amplifying or replicating a target nuclei acid strand also comprises replicating or amplifying a complementary strand of the target nucleic acid strand. Amplified products can be subjected to subsequence analyses, including but not limited to melting curve analysis, nucleotide sequencing, single-strand conformation polymorphism assay, allele-specific oligonucleotide hybridization, Southern blot analysis, and restriction endonuclease digestion.

The terms "hybridizes," and "annealing," as used herein, refer to a reaction in which one or more polynucleotides interact to form a complex that is stabilized via hydrogen bonding between the bases of the nucleotide residues. The hydrogen bonding can occur by Watson Crick base pairing, Hoogstein binding, or in any other sequence sensitive or specific manner. The complex can comprise two strands forming a duplex structure, three or more strands forming a multi stranded complex, a single self-hybridizing strand, or any combination of these. A hybridization reaction can constitute a step in a more extensive process, such as the initiation of a PCR, or the enzymatic cleavage of a polynucleotide by a ribozyme. A first sequence that can be stabilized via hydrogen bonding with the bases of the nucleotide residues of a second sequence can generally be "hybridizable" to the second sequence. In such a case, the second sequence can also be the to be hybridizable to the first sequence.

The terms "complement," "complements," "complementary," and "complementarity," as used herein, refer to a sequence that can hybridize to a given sequence. In some cases, a first sequence that is hybridizable to a second sequence or set of second sequences is specifically or selectively hybridizable to the second sequence or set of second sequences, such that hybridization to the second sequence or set of second sequences is used. Hybridizable sequences can share a degree of sequence complementarity over all or a portion of their respective lengths, such as between 25%-100% complementarity, including at least about 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, and 100% sequence complementarity.

As used in this specification and the appended claims, the singular forms "a," "an," and "the" include plural referents unless the content clearly dictates otherwise. The use of the words "a" or "an" when used in conjunction with the term "comprising" herein can mean "one," but it is also consistent with the meaning of "one or more," "at least one," and "one or more than one."

It should also be noted that the term "or" is generally employed in its sense including "and/or" unless the content clearly dictates otherwise.

Unless the context requires otherwise, throughout the specification and claims which follow, the word "comprise" and variations thereof, such as, "comprises," "including," and "comprising" are to be construed in an open, inclusive sense, that is, as "including, but not limited to."

The term "consisting of" refers to compositions, methods, and respective components thereof as described herein, which are exclusive of any element not recited in that description.

As used herein the term "consisting essentially of" refers to those elements that can be needed for a given method or system described herein. The term permits the presence of elements that do not materially affect or alter the basic and novel or functional characteristic(s) of that method or system (i.e. inactive or non-immunogenic ingredients) as described herein.

This disclosure is not limited by the methods and materials disclosed herein, and any methods and materials similar or equivalent to those described herein can be used in the practice or testing of embodiments of this disclosure. The terminology used herein is for the purpose of describing particular embodiments only, and is not intended to limit the scope of the present disclosure, which is defined solely by the claims.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments can perform functions in a different order, or functions can be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. Moreover, due to biological functional equivalency considerations, some changes can be made in protein structure without affecting the biological or chemical action in kind or amount. These and other changes can be made to the disclosure in light of the detailed description. All such modifications are intended to be included within the scope of the appended claims.

As used herein, the term "capable of when used with a verb, encompasses or means the action of the corresponding verb. For example, "capable of interacting" also means interacting, "capable of cleaving" also means cleaves, "capable of binding" also means binds and "capable of specifically targeting..." also means specifically targets.

Other definitions of terms can appear throughout the specification. Before the exemplary embodiments are described in more detail, it is to be understood that this disclosure is not limited to particular embodiments described, and as such can vary. It is also to be understood that the terminology used herein is for the purpose of describing particular embodiments only, and is not intended to be limiting, since the scope of the present disclosure will be defined only by the appended claims.

The term "about" when referring to a measurable value such as an amount, a temporal duration, and the like, is meant to encompass variations of ±10% from a specified value. As used herein, "about" and "approximately" generally mean an acceptable degree of error for the quantity measured given the nature or precision of the measurements.

Concentrations, amounts, volumes, percentages, and other numerical values can be presented herein in a range format. It is also to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited.

Ranges: throughout this disclosure, various aspects of the present disclosure can be presented in a range format. It should be understood that the description in range format is merely for convenience and brevity and should not be construed as an inflexible limitation on the scope of the present disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

Numeric ranges are inclusive of the numbers defining the range. Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges can independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

Unless otherwise defined, all technical and scientific terms used herein have the same meaning as commonly understood by one of ordinary skill in the art to which this disclosure belongs. Although methods and materials similar or equivalent to those described herein can be used in the practice or testing of the presently described methods, kit, and systems, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of this disclosure will be apparent from the following detailed description, and from the claims.

### BRIEF DESCRIPTION OF THE DRAWINGS

Various features described herein are set forth with particularity in the appended claims. A better understanding of the features and advantages of the disclosed subject matter can be obtained by reference to the following detailed description that sets forth exemplary embodiments, in which the disclosed principles are utilized, and the accompanying drawings.
**FIG. 1** is a schematic representation of a plurality of first primers binding to an integrated nucleic acid molecule.
**FIG. 2** is a schematic representation of a plurality of primers with a primer handle (PH2) binding to a strand product generated from a first primer.

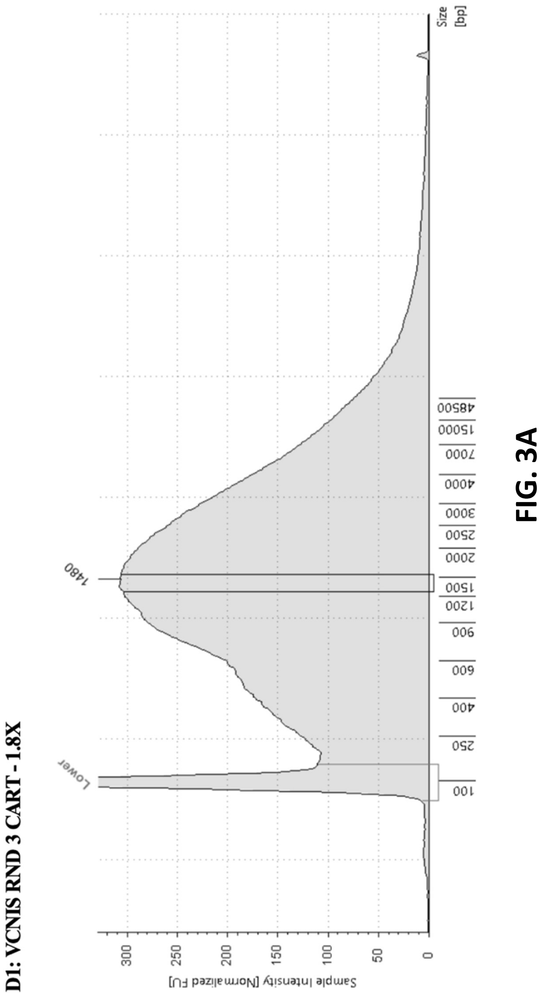

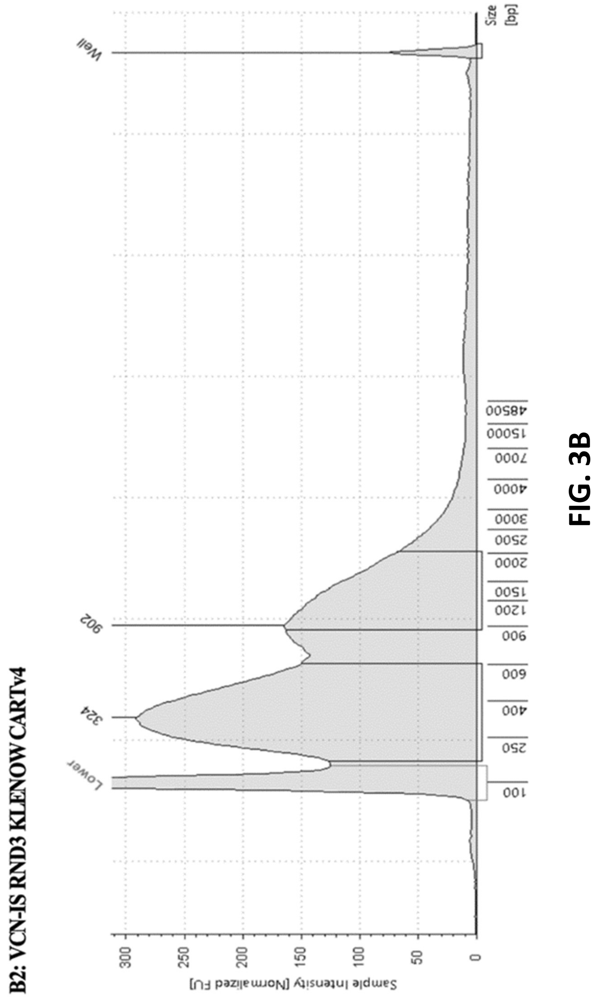

**FIGs. 3A-3B** are graphs that show the amplification product results of two amplification runs. The protocol of **FIG. 3A** led to longer amplicons than those from the protocol of **FIG. 3B****.** The difference between these two runs was the optimized cycling program - much shorter overall time due to simplified annealing temperature steps - changed from trying to land each tile individually in succession with pauses at each increment from 68-58°C to going straight to 58°C. This reduced the cycling time from ~7 hours to 1.5 hours. The enzyme used, *Thermodesulfatator indicus* exo- LF DNA polymerase, is extremely temperature tolerant and does not seem to disengage from the template even at 95°C. This results in a direct correlation of total amplification time to max length of the product.
**FIGs. 4A-4B** are graphs that show amplification product results of two amplification runs using the unnatural dNTP dideoxycytosine (ddCTP) either 100% (**FIG. 4A**) or 1% (**FIG. 4B**) of the cytosine nucleotides were replaced with the dideoxy analogue.
**FIGs. 5A-5B** are graphs that show amplification product results of two amplification runs using the unnatural dNTP deoxythymidine-(a-thio)-triphosphate (dTTPαS). Either 100% (**FIG. 5A**) or 1% (**FIG. 5B**) of the thymidine nucleotides were replaced with the alpha-thio analogue.
**FIGs. 6A-6F** are different graphs that show representative traces from prototype quality control assays with 20 ng CAR with gDNA. In these graphs, RND-1 = first amplification reaction with the forward primers, RND-2 = second strand synthesis, and RND-3 = final library amplification to complete sequencing adaptors.

### DETAILED DESCRIPTION

The present disclosure provides methods for determining a junction integration site of a nucleic acid molecule in a genome and a copy number of integration. In some aspects, the methods of the present disclosure include: (a) providing a sample of a genomic DNA molecule from a cell, wherein the genomic DNA molecule comprises an integrated nucleic acid molecule; (b) contacting a sequence of the integrated nucleic acid molecule with a first primer, wherein the first primer is configured to hybridize to the sequence of the integrated nucleic acid molecule, and wherein the first primer includes a first primer handle comprising a sequence that does not hybridize to a sequence of the integrated nucleic acid molecule; (c) extending the first primer to generate a first extension strand product comprising a sequence of the integrated nucleic acid molecule and a sequence of the genomic DNA molecule; (d) contacting the first extension strand product with a second primer, wherein the second primer is a random primer configured to anneal to the first extension strand product, and wherein the second primer includes a second primer handle that is configured to hybridize to an amplification primer; (e) extending the second primer to generate a second extension strand product; (f) amplifying the first extension strand product and the second extension strand product with a set of amplification primers to generate a junction-specific amplification product, wherein the set of amplification primers includes index adaptors; and (g) analyzing the junction-specific amplification product, wherein the analyzing includes: (i) identifying the junction integration site in the nucleic acid molecule in the genome, and (ii) identifying a copy number of integration, wherein the identifying the junction of integration and identifying a copy number of integration occur concurrently, and wherein the identifying the junction of integration and identifying a copy number of integration occur at a single cell resolution.

In some cases, the methods described herein can be agnostic to transduction strategies for a vector. In some cases, the methods described herein can improve the coverage, accuracy, and throughput of vector integration and copy number identification.

### Nucleic Acid Amplifications

The methods provided herein can be used for identification and quantification of vector copy number and integration sites. Amplification of integration loci can be accomplished using primers with various nucleic acid amplifications, including but not limited to, polymerase chain reactions and isothermal amplifications.

### Polymerase Chain Reactions

The nucleic acid amplification described herein can be a Polymerase Chain Reaction (PCR). A sample (e.g., DNA) containing the nucleic acid sequence to be amplified can be heated to denature or separate the two strands of the nucleic acid. The sample can then be cooled and mixed with specific oligonucleotide primers, allowing annealing of the primers to the single-stranded DNA template. Following this hybridization, a thermostable DNA polymerase in a reaction mixture can be added to the sample, along with free dNTPs that are linked by the polymerase to the replicating nucleic acid strand. After allowing polymerization to proceed to completion, the products can again be heated to separate the strands and subjected to another round of primer hybridization and polymerase replication. This process can be repeated any number of times. Since each nucleic acid product of a given cycle of this process can serve as a template for production of two new nucleic acid molecules (one from each parent strand), the PCR process can result in an exponential increase in the concentration of the target sequence. Thus, in a well-controlled, high-fidelity PCR process, as few as 20 cycles can result in an over one million fold amplification of the target nucleic acid sequence.

### Thermostable DNA Polymerases

In nucleic acid amplification techniques, thermostable DNA polymerases can be used to synthesize a new DNA strand complementary to the DNA template strand. The thermostable DNA polymerase most commonly used in PCR is Taq polymerase, isolated from the thermophilic bacterium Thermus aquaticus. Taq polymerase can function at temperatures of 70-80°C, and can maintain substantial activity upon repeated exposure to temperatures of 92°-95°C.

In addition to Taq polymerase, other thermostable polymerases can be used. Alternatives for Taq polymerase in PCR are known in the art and include polymerases isolated from the thermophilic bacteria *Thermus thermophilus* (Tth polymerase), *Thermococcus litoralis* (Tli or VENT^{™} polymerase), *Pyrococcus furiosus* (Pfu or DEEPVENT^{™} polymerase), *Pyrococcus woosii* (Pwo polymerase) and other *Pyrococcus* species, *Bacillus sterothermophilus* (Bst polymerase), *Sulfolobus acidocaldarius* (Sac polymerase), *Thermoplasma acidophilum* (Tac polymerase), *Thermus flavus* (Tfl/Tub polymerase), *Thermus ruber* (Tru polymerase), *Thermus brockianus* (DYNAZYME^{™} polymerase), *Thermotoga neapolitana* (Tne polymerase), *Thermotoga maritima* (Tma polymerase) and other species of the *Thermotoga* genus (Tsp polymerase) and *Methanobacterium thermoautotrophicum* (Mth polymerase).

### Isothermal Amplification

Isothermal amplification can rapidly and efficiently copy nucleic acids without temperature changing cycles. Instead, isothermal amplification can use specific DNA polymerases, and specially designed primer sets to exponentially amplify a target sequence. Different isothermal amplification methods can utilize different DNA polymerases. Helicase-dependent amplification (HDA) can use helicase to unwind DNA, allowing primers to bind. Two accessory proteins, MutL and single-stranded DNA-binding protein (SSB), can be used to prevent complimentary strands from associating. Isothermal multiple displacement amplification (IMDA) can use strand-displacing DNA polymerase and multiple primer sets. Particular advantages of IMDA can include increased sensitivity and specificity. Loop-mediated isothermal amplification (LAMP) can use 4-6 primers and a strand-displacing DNA polymerase. LAMP is a highly efficient amplification method that can synthesize a large quantity of DNA in a short period of time. Recombinase polymerase amplification (RPA) can use recombinase, primers, SSB, and a strand-displacing DNA polymerase to amplify DNA. Recombinase can be complexed with the primer and the complex then can use strand exchange to bind to double-stranded DNA. After the strand exchange an SSB T4 gp32 can stabilize the displaced strand. Rolling circle amplification (RCA) can synthesize long single-stranded DNA using a short, circular single-stranded DNA template and a single primer. RCA can use a strand-displacing DNA polymerase called phi 29. Single primer isothermal amplification (SPIA) can use only one DNA-RNA chimeric primer along with RNAase H and a strand-displacing DNA polymerase. Strand displacement amplification can rely on a restriction enzyme (HincII) and an exonuclease-deficient DNA polymerase. HinCII can nick the target DNA, and the DNA polymerase can then extend the 3' end.

### Methods for Analyzing a Cell

In some aspects, provided herein are methods for the identification and quantification of a copy number and integration site of a vector.

The method for determining a junction integration site of a nucleic acid molecule in a genome and a copy number of integration can include providing a sample of a genomic DNA molecule from a cell, wherein the genomic DNA molecule includes an integrated nucleic acid molecule. The method can further include contacting a sequence of the integrated nucleic acid molecule with a first primer. The first primer can be configured to hybridize to the sequence of the integrated nucleic acid molecule. In some cases, the first primer can include a first primer handle having a sequence that does not hybridize to a sequence of the integrated nucleic acid molecule. The method can further include extending the first primer to generate a first extension strand product having a sequence of the integrated nucleic acid molecule and a sequence of the genomic DNA molecule. In some cases, a first extension strand product can be generated using a nucleic acid amplification. A nucleic acid amplification can be or include polymerase chain reaction (PCR) or an isothermal amplification. The method can further include contacting the first extension strand product with a second primer. The second primer can be or include a random primer. In some cases, the second primer can be a random primer configured to anneal to the first extension strand product. The second primer can include a second primer handle that is configured to hybridize to an amplification primer.

The method can further include extending the second primer to generate a second extension strand product. In some cases, a second extension strand product can be generated using a nucleic acid amplification. A nucleic acid amplification can be or include a polymerase chain reaction (PCR) or an isothermal amplification. The method can further include amplifying the first extension strand product and the second extension strand product with a set of amplification primers to generate a junction-specific amplification product. The amplification primers can be index adaptors. The method can further include analyzing the junction-specific amplification product. The junction-specific amplification product (e.g., amplicon) can be sequenced. Sequencing can be or include next generation sequencing. The junction-specific amplification product can be analyzed to identify a copy number of integration for the integrated nucleic acid molecule (e.g., vector). In some cases, the junction-specific amplification product can be analyzed to identify an integration site in the nucleic acid molecule in the genome of the integrated nucleic acid molecule. In some cases, the analysis is performed such that identifying an integration site and identifying a copy number of integration can be determined simultaneously. In some cases, analysis of the junction-specific amplification product can be performed at a single cell resolution. Without wishing to be bound by theory, analyzing a transcriptome of a single cell at a time can allow for better characterization of the heterogeneity of a sample.

A nucleic acid molecule can be integrated into a cell genome using different transduction methods (e.g., virus-mediated transfer of the nucleic acid molecule into the cell). These can include, but are not limited to, adenoviral transduction and lentiviral transduction. In lentiviral vector transduction, a virus can enter the cell and viral RNA can be transcribed by the reverse transcriptase to produce double-stranded DNA. The double-stranded can enter the nucleus where a target transgene can be integrated into a host genome via lentiviral integrase enzymes.

The vector can include an integrated nucleic acid molecule that encodes a payload. In some cases, the payload can be a diagnostic imaging agent, such as a contrast agent. In some cases, the payload includes a therapeutic agent, including, but not limited to, a nuclease, a recombinase, a growth factor, an antibody, a chimeric antigen receptor, a T cell receptor, a cytokine, a cytokine inhibitor or agonist, a transcription factor, an organelle, a nucleic acid molecule, a therapeutic DNA, a therapeutic RNA, a retrotransposon, a reverse transcriptase, an oligonucleotide, an aptazyme, an aptamer, or a ribozyme, a generic or specific kinase inhibitor, a small molecule drug, an immunomodulator, a tumor suppressor, a developmental regulator, a cancer vaccine, an anesthetic, an enzyme, a hormone, a ligand, a receptor, a T cell receptor, a transposon, a retrotransposon, a DNA polymerase, a RNA dependent DNA polymerase, a homing endonuclease, interferons, chemokines, insulin, growth factors, an antisense oligonucleotide, an RNAi, a shRNA, and any combination thereof. The payload can be a prophylactic agent. In some cases, the payload comprises a biomarker. The payload can also comprise an exogenous antigen or an enzyme. In some cases, the payload comprises a metabolite molecule. In some cases, the payload comprises a lipid molecule. In some cases, the payload comprises a structural protein. In some cases, the payload comprises a hormone or a hormonal protein.

In some cases, the payload can comprise a chimeric antigen receptor (CAR).

In some aspects, the method includes: (a) providing a sample of a genomic DNA molecule from a cell, wherein the genomic DNA molecule includes an integrated nucleic acid molecule; (b) contacting a sequence of the integrated nucleic acid molecule with a first primer, wherein the first primer is configured to hybridize to the sequence of the integrated nucleic acid molecule, and wherein the first primer includes a first primer handle comprising a sequence that does not hybridize to a sequence of the integrated nucleic acid molecule; (c) extending the first primer to generate a first extension strand product including a sequence of the integrated nucleic acid molecule and a sequence of the genomic DNA molecule; (d) contacting the first extension strand product with a second primer, wherein the second primer is a random primer configured to anneal to the first extension strand product, and wherein the second primer includes a second primer handle that is configured to hybridize to an amplification primer; (e) extending the second primer to generate a second extension strand product; (f) amplifying the first extension strand product and the second extension strand product with a set of amplification primers to generate a junction-specific amplification product, wherein the set of amplification primers includes index adaptors; and (g) analyzing the junction-specific amplification product. The analyzing of the junction-specific amplification product can include (i) identifying the junction integration site in the nucleic acid molecule in the genome, and (ii) identifying a copy number of integration.

In some embodiments, identifying the junction of integration and identifying a copy number of integration occur concurrently. In some embodiments, identifying the junction of integration and identifying a copy number of integration occur sequentially. In some embodiments, identifying the junction of integration and identifying a copy number of integration occur at a single cell resolution.

The method for determining a junction integration site of a nucleic acid molecule in a genome and a copy number of integration can include providing a sample of a genomic DNA molecule from a cell, wherein the genomic DNA molecule includes an integrated nucleic acid molecule. In some embodiments, the sample includes a biological sample. In some embodiments, the sample includes a purified sample. In some embodiments, the biological sample includes a target nucleic acid molecule subject to the methods of copy number and integration site identification provided herein. In some embodiments, a cell of the present disclosure can include a T cell, an NKT cell, an NK cell, or a precursor thereof.

The method can further include contacting a sequence of the integrated nucleic acid molecule with a first primer. Amplification of an integration loci of a vector can be accomplished with a first primer. In some cases, the method includes a plurality of first primers. In some cases, a first primer can hybridize to at least a portion of an integrated nucleic acid molecule. In some cases, a plurality of first primers can anneal to different sequences of the integrated nucleic acid molecule. In some cases, a plurality of first primers can anneal to distinct sequences of the integrated nucleic acid molecule. The first primer can bind upstream of a 3' junction of the integrated nucleic acid molecule and genomic DNA molecule of the cell. In some cases, the first primer can bind at least about 1 base pair, at least about 5 base pairs, at least about 10 base pairs, at least about 50 base pairs, at least about 100 base pairs, at least about 125 base pairs, at least about 150 base pairs, at least about 175 base pairs, at least about 200 base pairs, at least about 250 base pairs, at least about 300 base pairs, at least about 400 base pairs, or at least about 500 base pairs upstream from the 3' junction of the integrated nucleic acid molecule and genomic DNA molecule. In some cases, the first primer can bind to at most about 500 base pairs, at most about 400 base pairs, at most about 300 base pairs, at most about 250 base pairs, at most about 200 base pairs, at most about 175 base pairs, at most about 150 base pairs, at most about 125 base pairs, at most about 100 base pairs, at most about 50 base pairs, at most about 10 base pairs, at most about 5 base pairs, or at most about 1 base pair upstream from the 3' junction of the integrated nucleic acid molecule and genomic DNA molecule.

The methods described herein can include a number of first primers. In some cases, the method includes at least about 2, at least about 3, at least about 4, at least about 5, at least about 6, at least about 7, at least about 8, at least about 9, or at least about 10 first primers. In some cases, the method comprises at most about 10, at most about 9, at most about 8, at most about 7, at most about 6, at most about 5, at most about 4, at most about 3, or at most about 2 first primers. In some cases, the method includes from about 2 first primers to about 12 first primers. In some cases, the method includes from about 2 first primers to about 3 first primers, about 2 first primers to about 4 first primers, about 2 first primers to about 5 first primers, about 2 first primers to about 6 first primers, about 2 first primers to about 7 first primers, about 2 first primers to about 8 first primers, about 2 first primers to about 9 first primers, about 2 first primers to about 10 first primers, about 2 first primers to about 11 first primers, about 2 first primers to about 12 first primers, about 3 first primers to about 4 first primers, about 3 first primers to about 5 first primers, about 3 first primers to about 6 first primers, about 3 first primers to about 7 first primers, about 3 first primers to about 8 first primers, about 3 first primers to about 9 first primers, about 3 first primers to about 10 first primers, about 3 first primers to about 11 first primers, about 3 first primers to about 12 first primers, about 4 first primers to about 5 first primers, about 4 first primers to about 6 first primers, about 4 first primers to about 7 first primers, about 4 first primers to about 8 first primers, about 4 first primers to about 9 first primers, about 4 first primers to about 10 first primers, about 4 first primers to about 11 first primers, about 4 first primers to about 12 first primers, about 5 first primers to about 6 first primers, about 5 first primers to about 7 first primers, about 5 first primers to about 8 first primers, about 5 first primers to about 9 first primers, about 5 first primers to about 10 first primers, about 5 first primers to about 11 first primers, about 5 first primers to about 12 first primers, about 6 first primers to about 7 first primers, about 6 first primers to about 8 first primers, about 6 first primers to about 9 first primers, about 6 first primers to about 10 first primers, about 6 first primers to about 11 first primers, about 6 first primers to about 12 first primers, about 7 first primers to about 8 first primers, about 7 first primers to about 9 first primers, about 7 first primers to about 10 first primers, about 7 first primers to about 11 first primers, about 7 first primers to about 12 first primers, about 8 first primers to about 9 first primers, about 8 first primers to about 10 first primers, about 8 first primers to about 11 first primers, about 8 first primers to about 12 first primers, about 9 first primers to about 10 first primers, about 9 first primers to about 11 first primers, about 9 first primers to about 12 first primers, about 10 first primers to about 11 first primers, about 10 first primers to about 12 first primers, or about 11 first primers to about 12 first primers.

In some cases, the first primer can bind from about 1 base pair to about 250 base pairs upstream from the 3' junction of the integrated nucleic acid molecule and genomic DNA molecule. In some cases, the first primer can bind from about 1 base pair to about 5 base pairs, about 1 base pair to about 10 base pairs, about 1 base pair to about 25 base pairs, about 1 base pair to about 50 base pairs, about 1 base pair to about 75 base pairs, about 1 base pair to about 100 base pairs, about 1 base pair to about 125 base pairs, about 1 base pair to about 150 base pairs, about 1 base pair to about 175 base pairs, about 1 base pair to about 200 base pairs, about 1 base pair to about 250 base pairs, about 5 base pairs to about 10 base pairs, about 5 base pairs to about 25 base pairs, about 5 base pairs to about 50 base pairs, about 5 base pairs to about 75 base pairs, about 5 base pairs to about 100 base pairs, about 5 base pairs to about 125 base pairs, about 5 base pairs to about 150 base pairs, about 5 base pairs to about 175 base pairs, about 5 base pairs to about 200 base pairs, about 5 base pairs to about 250 base pairs, about 10 base pairs to about 25 base pairs, about 10 base pairs to about 50 base pairs, about 10 base pairs to about 75 base pairs, about 10 base pairs to about 100 base pairs, about 10 base pairs to about 125 base pairs, about 10 base pairs to about 150 base pairs, about 10 base pairs to about 175 base pairs, about 10 base pairs to about 200 base pairs, about 10 base pairs to about 250 base pairs, about 25 base pairs to about 50 base pairs, about 25 base pairs to about 75 base pairs, about 25 base pairs to about 100 base pairs, about 25 base pairs to about 125 base pairs, about 25 base pairs to about 150 base pairs, about 25 base pairs to about 175 base pairs, about 25 base pairs to about 200 base pairs, about 25 base pairs to about 250 base pairs, about 50 base pairs to about 75 base pairs, about 50 base pairs to about 100 base pairs, about 50 base pairs to about 125 base pairs, about 50 base pairs to about 150 base pairs, about 50 base pairs to about 175 base pairs, about 50 base pairs to about 200 base pairs, about 50 base pairs to about 250 base pairs, about 75 base pairs to about 100 base pairs, about 75 base pairs to about 125 base pairs, about 75 base pairs to about 150 base pairs, about 75 base pairs to about 175 base pairs, about 75 base pairs to about 200 base pairs, about 75 base pairs to about 250 base pairs, about 100 base pairs to about 125 base pairs, about 100 base pairs to about 150 base pairs, about 100 base pairs to about 175 base pairs, about 100 base pairs to about 200 base pairs, about 100 base pairs to about 250 base pairs, about 125 base pairs to about 150 base pairs, about 125 base pairs to about 175 base pairs, about 125 base pairs to about 200 base pairs, about 125 base pairs to about 250 base pairs, about 150 base pairs to about 175 base pairs, about 150 base pairs to about 200 base pairs, about 150 base pairs to about 250 base pairs, about 175 base pairs to about 200 base pairs, about 175 base pairs to about 250 base pairs, or about 200 base pairs to about 250 base pairs upstream from the 3' junction of the integrated nucleic acid molecule and genomic DNA molecule.

In some cases, the first primer can be a hybrid molecule including a primer handle. The first primer can include a first primer handle, including a fixed 5' region that may not bind to the integrated nucleic acid molecule or the genomic DNA molecule.

In some cases, the first primer handle is biotinylated. Without wishing to be bound by theory, biotinylation of the first primer handle can enrich amplification products. In some cases, the primer is tagged with biotin or 6-carboxyfluorescein (FAM) for visualization on a lateral flow immunoassay strip. The first primer can also include a barcode sequence and/or a hybridization tag (e.g., hybridization probe). The hybridization tag can include a fragment of DNA or RNA. The hybridization tag can be at least about, at most about, or about 15 nucleotides, 50 nucleotides, 100 nucleotides, 250 nucleotides, 500 nucleotides, 750 nucleotides, 1000 nucleotides, 1500 nucleotides, 2000 nucleotides, or 5000 nucleotides in length. A hybridization tag can be radioactively or fluorescently labeled to detect the presence of nucleotide sequences in analyzed DNA or RNA. Examples of hybridization probes include, but are not limited to, Scorpion^{®} probes, Molecular Beacon probes, TagMan^{®} probes, Locked Nucleic Acid (LNA^{®}) probes, and Cycling Probe Technology (CPT).

Unique molecular identifiers (UMIs) are an additional form of molecular barcoding that can assist in error correction and improve accuracy during sequencing methods. In some cases, the first primers described herein can comprise a unique molecular identifier (UMI). In some cases, the first primer can be combinatorially barcoded on a cleavable linker attached to a microparticle. In some cases, the first primer can be combinatorially barcoded on a cleavable linker attached to another solid phase material.

In some cases, the first primer includes a number of degenerate nucleotides. A "degenerate nucleotide" refers to a nucleotide that can function similarly or yield a similar output as a structurally different nucleotide. In some cases, a first primer includes at least about 2 degenerate nucleotides, at least about 3 degenerate nucleotides, at least about 4 degenerate nucleotides, at least about 5 degenerate nucleotides, at least about 6 degenerate nucleotides, at least about 7 degenerate nucleotides, at least about 8 degenerate nucleotides, at least about 9 degenerate nucleotides, at least about 10 degenerate nucleotides, or at least about 12 degenerate nucleotides. In some cases, a first primer comprises about 1 degenerate nucleotide to about 12 degenerate nucleotides. In some cases, a first primer comprises about 1 degenerate nucleotide to about 2 degenerate nucleotides, about 1 degenerate nucleotide to about 3 degenerate nucleotides, about 1 degenerate nucleotide to about 4 degenerate nucleotides, about 1 degenerate nucleotide to about 5 degenerate nucleotides, about 1 degenerate nucleotide to about 6 degenerate nucleotides, about 1 degenerate nucleotide to about 7 degenerate nucleotides, about 1 degenerate nucleotide to about 8 degenerate nucleotides, about 1 degenerate nucleotide to about 9 degenerate nucleotides, about 1 degenerate nucleotide to about 10 degenerate nucleotides, about 1 degenerate nucleotide to about 11 degenerate nucleotides, about 1 degenerate nucleotide to about 12 degenerate nucleotides, about 2 degenerate nucleotides to about 3 degenerate nucleotides, about 2 degenerate nucleotides to about 4 degenerate nucleotides, about 2 degenerate nucleotides to about 5 degenerate nucleotides, about 2 degenerate nucleotides to about 6 degenerate nucleotides, about 2 degenerate nucleotides to about 7 degenerate nucleotides, about 2 degenerate nucleotides to about 8 degenerate nucleotides, about 2 degenerate nucleotides to about 9 degenerate nucleotides, about 2 degenerate nucleotides to about 10 degenerate nucleotides, about 2 degenerate nucleotides to about 11 degenerate nucleotides, about 2 degenerate nucleotides to about 12 degenerate nucleotides, about 3 degenerate nucleotides to about 4 degenerate nucleotides, about 3 degenerate nucleotides to about 5 degenerate nucleotides, about 3 degenerate nucleotides to about 6 degenerate nucleotides, about 3 degenerate nucleotides to about 7 degenerate nucleotides, about 3 degenerate nucleotides to about 8 degenerate nucleotides, about 3 degenerate nucleotides to about 9 degenerate nucleotides, about 3 degenerate nucleotides to about 10 degenerate nucleotides, about 3 degenerate nucleotides to about 11 degenerate nucleotides, about 3 degenerate nucleotides to about 12 degenerate nucleotides, about 4 degenerate nucleotides to about 5 degenerate nucleotides, about 4 degenerate nucleotides to about 6 degenerate nucleotides, about 4 degenerate nucleotides to about 7 degenerate nucleotides, about 4 degenerate nucleotides to about 8 degenerate nucleotides, about 4 degenerate nucleotides to about 9 degenerate nucleotides, about 4 degenerate nucleotides to about 10 degenerate nucleotides, about 4 degenerate nucleotides to about 11 degenerate nucleotides, about 4 degenerate nucleotides to about 12 degenerate nucleotides, about 5 degenerate nucleotides to about 6 degenerate nucleotides, about 5 degenerate nucleotides to about 7 degenerate nucleotides, about 5 degenerate nucleotides to about 8 degenerate nucleotides, about 5 degenerate nucleotides to about 9 degenerate nucleotides, about 5 degenerate nucleotides to about 10 degenerate nucleotides, about 5 degenerate nucleotides to about 11 degenerate nucleotides, about 5 degenerate nucleotides to about 12 degenerate nucleotides, about 6 degenerate nucleotides to about 7 degenerate nucleotides, about 6 degenerate nucleotides to about 8 degenerate nucleotides, about 6 degenerate nucleotides to about 9 degenerate nucleotides, about 6 degenerate nucleotides to about 10 degenerate nucleotides, about 6 degenerate nucleotides to about 11 degenerate nucleotides, about 6 degenerate nucleotides to about 12 degenerate nucleotides, about 7 degenerate nucleotides to about 8 degenerate nucleotides, about 7 degenerate nucleotides to about 9 degenerate nucleotides, about 7 degenerate nucleotides to about 10 degenerate nucleotides, about 7 degenerate nucleotides to about 11 degenerate nucleotides, about 7 degenerate nucleotides to about 12 degenerate nucleotides, about 8 degenerate nucleotides to about 9 degenerate nucleotides, about 8 degenerate nucleotides to about 10 degenerate nucleotides, about 8 degenerate nucleotides to about 11 degenerate nucleotides, about 8 degenerate nucleotides to about 12 degenerate nucleotides, about 9 degenerate nucleotides to about 10 degenerate nucleotides, about 9 degenerate nucleotides to about 11 degenerate nucleotides, about 9 degenerate nucleotides to about 12 degenerate nucleotides, about 10 degenerate nucleotides to about 11 degenerate nucleotides, about 10 degenerate nucleotides to about 12 degenerate nucleotides, or about 11 degenerate nucleotides to about 12 degenerate nucleotides.

In some embodiments, the primer is at least about 3 nucleotides, at least about 5 nucleotides, at least about 10 nucleotides, at least about 15 nucleotides, at least about 20 nucleotides, at least about 25 nucleotides, at least about 30 nucleotides, at least about 35 nucleotides, at least about 40 nucleotides, at least about 45 nucleotides, at least about 50 nucleotides, at least about 60 nucleotides, at least about 70 nucleotides, at least about 80 nucleotides, at least about 90 nucleotides, at least about 100 nucleotides, at least about 150 nucleotides, or at least about 200 nucleotides in length. In some embodiments, the primer is at most about 200 nucleotides, at most about 150 nucleotides, at most about 100 nucleotides, at most about 90 nucleotides, at most about 80 nucleotides, at most about 70 nucleotides, at most about 60 nucleotides, at most about 50 nucleotides, at most about 45 nucleotides, at most about 40 nucleotides, at most about 35 nucleotides, at most about 30 nucleotides, at most about 25 nucleotides, at most about 20 nucleotides, at most about 15 nucleotides, at most about 10 nucleotides, at most about 5 nucleotides, or at most about 3 nucleotides in length.

In some embodiments, the primer is about 3 nucleotides to about 100 nucleotides in length. In some embodiments, the primer is at most about 100 nucleotides. In some embodiments, the primer is about 3 nucleotides to about 5 nucleotides, about 3 nucleotides to about 10 nucleotides, about 3 nucleotides to about 20 nucleotides, about 3 nucleotides to about 30 nucleotides, about 3 nucleotides to about 40 nucleotides, about 3 nucleotides to about 50 nucleotides, about 3 nucleotides to about 60 nucleotides, about 3 nucleotides to about 70 nucleotides, about 3 nucleotides to about 80 nucleotides, about 3 nucleotides to about 90 nucleotides, about 3 nucleotides to about 100 nucleotides, about 5 nucleotides to about 10 nucleotides, about 5 nucleotides to about 20 nucleotides, about 5 nucleotides to about 30 nucleotides, about 5 nucleotides to about 40 nucleotides, about 5 nucleotides to about 50 nucleotides, about 5 nucleotides to about 60 nucleotides, about 5 nucleotides to about 70 nucleotides, about 5 nucleotides to about 80 nucleotides, about 5 nucleotides to about 90 nucleotides, about 5 nucleotides to about 100 nucleotides, about 10 nucleotides to about 20 nucleotides, about 10 nucleotides to about 30 nucleotides, about 10 nucleotides to about 40 nucleotides, about 10 nucleotides to about 50 nucleotides, about 10 nucleotides to about 60 nucleotides, about 10 nucleotides to about 70 nucleotides, about 10 nucleotides to about 80 nucleotides, about 10 nucleotides to about 90 nucleotides, about 10 nucleotides to about 100 nucleotides, about 20 nucleotides to about 30 nucleotides, about 20 nucleotides to about 40 nucleotides, about 20 nucleotides to about 50 nucleotides, about 20 nucleotides to about 60 nucleotides, about 20 nucleotides to about 70 nucleotides, about 20 nucleotides to about 80 nucleotides, about 20 nucleotides to about 90 nucleotides, about 20 nucleotides to about 100 nucleotides, about 30 nucleotides to about 40 nucleotides, about 30 nucleotides to about 50 nucleotides, about 30 nucleotides to about 60 nucleotides, about 30 nucleotides to about 70 nucleotides, about 30 nucleotides to about 80 nucleotides, about 30 nucleotides to about 90 nucleotides, about 30 nucleotides to about 100 nucleotides, about 40 nucleotides to about 50 nucleotides, about 40 nucleotides to about 60 nucleotides, about 40 nucleotides to about 70 nucleotides, about 40 nucleotides to about 80 nucleotides, about 40 nucleotides to about 90 nucleotides, about 40 nucleotides to about 100 nucleotides, about 50 nucleotides to about 60 nucleotides, about 50 nucleotides to about 70 nucleotides, about 50 nucleotides to about 80 nucleotides, about 50 nucleotides to about 90 nucleotides, about 50 nucleotides to about 100 nucleotides, about 60 nucleotides to about 70 nucleotides, about 60 nucleotides to about 80 nucleotides, about 60 nucleotides to about 90 nucleotides, about 60 nucleotides to about 100 nucleotides, about 70 nucleotides to about 80 nucleotides, about 70 nucleotides to about 90 nucleotides, about 70 nucleotides to about 100 nucleotides, about 80 nucleotides to about 90 nucleotides, about 80 nucleotides to about 100 nucleotides, or about 90 nucleotides to about 100 nucleotides in length.

The method can further include extending the first primer to generate a first extension strand product comprising a sequence of the integrated nucleic acid molecule and a sequence of the genomic DNA molecule. The first primer or plurality of first primers can be separated in annealing temperature by 1°C. Temperature separation of the primers can allow for multiple primer annealing to sequences of the integrated nucleic acid molecule during a nucleic acid amplification. The nucleic acid amplification can be a temperature gradient PCR. In some cases, a temperature is changed over the course of the nucleic acid amplification. In some cases, the nucleic acid amplification comprises thermocycling the sample and/or primer. In some cases, the nucleic acid amplification comprises keeping the sample at a constant temperature during the amplification. During a nucleic acid amplification with the first primer or plurality of first primers, a thermostable enzyme can be used to extend the first primer. In some cases, the thermostable enzyme can have strand displacement activity. In some cases, the thermostable enzyme includes a *thermodesulfatator indicus* (exo-) DNA polymerase. In some cases, the thermostable enzyme includes a *Bacillus stearothermophilus* polymerase, a large fragment of a *Bacillus stearothermophilus* polymerase, a exo-Klenow polymerase, a Bst 2.0 polymerase, a Bst 3.0 polymerase, a SD DNA polymerase, a phi29 DNA polymerase, a sequencing-grade T7 exo-polymerase, a *Thermus aquaticus* (*e.g.,* Taq-polA), a *Thermotoga maritima* (*e.g.,* Tma-polA), a Pfu-polB, a Pab-polB, an OmniTaq 2 LA DNA polymerase, or any mutants thereof.

In some cases, extending the first primer includes heating the sample at a constant temperature for a duration of time. In some cases, extending the first primer includes heating the sample at a cyclic temperature for a duration of time. The cyclic temperature can include heating the sample to a first temperature, cooling down the sample, and heating the sample to a second temperature after cooling down. The cyclic temperature can include a range of temperature. In some cases, the constant temperature includes at least about 30°C, at least about 40°C, at least about 50°C, at least about 60°C, at least about 70°C, at least about 75°C, at least about 80°C, at least about 85°C, at least about 90°C, at least about 91°C, at least about 92°C, at least about 93°C, at least about 94°C, at least about 95°C, at least about 96°C, at least about 97°C, at least about 98°C, at least about 99°C, at least about 100°C, at least about 105°C, at least about 110°C, at least about 115°C, at least about 120°C, at least about 125°C, at least about 130°C, at least about 140°C, or at least about 150°C. In some cases, the constant temperature comprises at most about 150°C, at most about 140°C, at most about 130°C, at most about 125°C, at most about 120°C, at most about 115°C, at most about 100°C, at most about 105°C, at most about 100°C, at most about 99°C, at most about 98°C, at most about 97°C, at most about 96°C, at most about 95°C, at most about 94°C, at most about 93°C, at most about 92°C, at most about 91°C, at most about 90°C, at most about 85°C, at most about 80°C, at most about 75°C, at most about 70°C, at most about 60°C, at most about 50°C, at most about 40°C, or at most about 30°C.

In some cases, the cyclic temperature can be from about 30°C to about 120°C. In some embodiments, the cyclic temperature is about 30°C to about 40°C, about 30°C to about 50°C, about 30°C to about 60°C, about 30°C to about 70°C, about 30°C to about 80°C, about 30°C to about 85°C, about 30°C to about 90°C, about 30°C to about 95°C, about 30°C to about 100°C, about 30°C to about 110°C, about 30°C to about 120°C, about 40°C to about 50°C, about 40°C to about 60°C, about 40°C to about 70°C, about 40°C to about 80°C, about 40°C to about 85°C, about 40°C to about 90°C, about 40°C to about 95°C, about 40°C to about 100°C, about 40°C to about 110°C, about 40°C to about 120°C, about 50°C to about 60°C, about 50°C to about 70°C, about 50°C to about 80°C, about 50°C to about 85°C, about 50°C to about 90°C, about 50°C to about 95°C, about 50°C to about 100°C, about 50°C to about 110°C, about 50°C to about 120°C, about 60°C to about 70°C, about 60°C to about 80°C, about 60°C to about 85°C, about 60°C to about 90°C, about 60°C to about 95°C, about 60°C to about 100°C, about 60°C to about 110°C, about 60°C to about 120°C, about 70°C to about 80°C, about 70°C to about 85°C, about 70°C to about 90°C, about 70°C to about 95°C, about 70°C to about 100°C, about 70°C to about 110°C, about 70°C to about 120°C, about 80°C to about 85°C, about 80°C to about 90°C, about 80°C to about 95°C, about 80°C to about 100°C, about 80°C to about 110°C, about 80°C to about 120°C, about 85°C to about 90°C, about 85°C to about 95°C, about 85°C to about 100°C, about 85°C to about 110°C, about 85°C to about 120°C, about 90°C to about 95°C, about 90°C to about 100°C, about 90°C to about 110°C, about 90°C to about 120°C, about 95°C to about 100°C, about 95°C to about 110°C, about 95°C to about 120°C, about 100°C to about 110°C, about 100°C to about 120°C, or about 110°C to about 120°C.

In some embodiments, the cyclic temperature can be from about 80°C to about 83°C, about 80°C to about 85°C, about 80°C to about 87°C, about 80°C to about 90°C, about 80°C to about 93°C, about 80°C to about 95°C, about 80°C to about 97°C, about 80°C to about 100°C, about 80°C to about 105°C, about 80°C to about 110°C, about 80°C to about 115°C, about 83°C to about 85°C, about 83°C to about 87°C, about 83°C to about 90°C, about 83°C to about 93°C, about 83°C to about 95°C, about 83°C to about 97°C, about 83°C to about 100°C, about 83°C to about 105°C, about 83°C to about 110°C, about 83°C to about 115°C, about 85°C to about 87°C, about 85°C to about 90°C, about 85°C to about 93°C, about 85°C to about 95°C, about 85°C to about 97°C, about 85°C to about 100°C, about 85°C to about 105°C, about 85°C to about 110°C, about 85°C to about 115°C, about 87°C to about 90°C, about 87°C to about 93°C, about 87°C to about 95°C, about 87°C to about 97°C, about 87°C to about 100°C, about 87°C to about 105°C, about 87°C to about 110°C, about 87°C to about 115°C, about 90°C to about 93°C, about 90°C to about 95°C, about 90°C to about 97°C, about 90°C to about 100°C, about 90°C to about 105°C, about 90°C to about 110°C, about 90°C to about 115°C, about 93°C to about 95°C, about 93°C to about 97°C, about 93°C to about 100°C, about 93°C to about 105°C, about 93°C to about 110°C, about 93°C to about 115°C, about 95°C to about 97°C, about 95°C to about 100°C, about 95°C to about 105°C, about 95°C to about 110°C, about 95°C to about 115°C, about 97°C to about 100°C, about 97°C to about 105°C, about 97°C to about 110°C, about 97°C to about 115°C, about 100°C to about 105°C, about 100°C to about 110°C, about 100°C to about 115°C, about 105°C to about 110°C, about 105°C to about 115°C, or about 110°C to about 115°C.

In some cases, extending the first primer includes contacting the first primer and/or the sample with deoxynucleoside triphosphates (dNTPs). The dNTPs can be natural or unnatural dNTPs. In some cases, the dNTPs comprise dATP, dCTP, dGTP, dTTP, and/or dUTP. In some cases, the unnatural dNTPs comprise dideoxynucleotide triphosphates (ddNTPs). The ddNTPs can comprise ddGTP, ddATP, ddTTP and ddCTP. The unnatural dNTPs can comprise α-thiol dNTPs (e.g., S-dNTPs). S-dNTPS can comprise dATPαS, dCTPαS, dGTPαS, and/or dTTPαS.

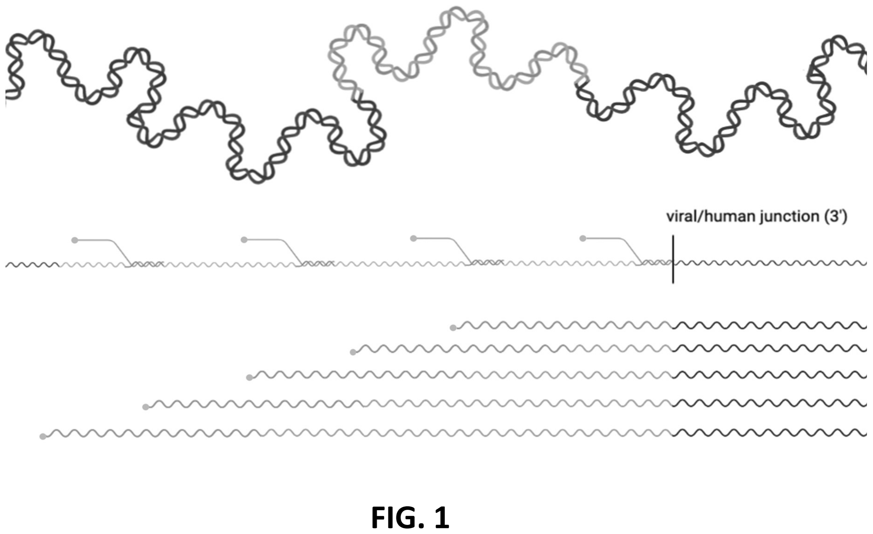

Without wishing to be bound by theory, thermal cycling with natural or unnatural dNTPs as described herein can be used to limit a length of a first extension strand product. Each first primer can be extended across the 3' junction of the integrated nucleic acid molecule into the adjacent genomic DNA molecule (**FIG. 1**). In some cases, the first extension strand product can comprise a bead (*e.g*., magnetic bead).

The method can further include contacting the first extension strand product with a second primer. The second primer can be a random primer comprising a random nucleotide sequence. In some cases, the second primer includes a number of degenerate nucleotides. In some cases, the second primer includes at least about 2 degenerate nucleotides, at least about 3 degenerate nucleotides, at least about 4 degenerate nucleotides, at least about 5 degenerate nucleotides, at least about 6 degenerate nucleotides, at least about 7 degenerate nucleotides, at least about 8 degenerate nucleotides, at least about 9 degenerate nucleotides, at least about 10 degenerate nucleotides, or at least about 12 degenerate nucleotides. The second primer can also include a barcode sequence and/or a hybridization tag (*e.g*., hybridization probe). The hybridization tag can include a fragment of DNA or RNA. The hybridization tag can be at least about, at most about, or about 15 nucleotides, 50 nucleotides, 100 nucleotides, 250 nucleotides, 500 nucleotides, 750 nucleotides, 1000 nucleotides, 1500 nucleotides, 2000 nucleotides, or 5000 nucleotides in length. In some cases, the first primers described herein can include a unique molecular identifier (UMI).

The methods described herein can include a second primer. In some cases, the methods described herein include a plurality of second primers. In some cases, the second primer comprises a fixed 5' region and the fixed 5' region can serve as a second primer handle. The second primer can end with a number of random nucleotides. In some cases, the second primer ends with at least about 3, at least about 4, at least about 5, at least about 6, at least about 7, at least about 8, at least about 9, at least about 10, at least about 11, or at least about 12 random nucleotides. In some cases, the second primer ends with at most 12 random nucleotides, at most about 11 random nucleotides, at most 10 random nucleotides at most 9 random nucleotides at most 8 random nucleotides at most 7 random nucleotides at most 6 random nucleotides at most 5 random nucleotides at most 4 random nucleotides or at most 3 random nucleotides.

The methods described herein can include a number of second primers. In some cases, the method comprises at least about 2, at least about 3, at least about 4, at least about 5, at least about 6, at least about 7, at least about 8, at least about 9, or at least about 10 first primers. In some cases, the method comprises at most about 10, at most about 9, at most about 8, at most about 7, at most about 6, at most about 5, at most about 4, at most about 3, or at most about 2 second primers. In some cases, the method comprises from about 2 second primers to about 12 second primers. In some cases, the method comprises from about 2 second primers to about 3 second primers, about 2 second primers to about 4 second primers, about 2 second primers to about 5 second primers, about 2 second primers to about 6 second primers, about 2 second primers to about 7 second primers, about 2 second primers to about 8 second primers, about 2 second primers to about 9 second primers, about 2 second primers to about 10 second primers, about 2 second primers to about 11 second primers, about 2 second primers to about 12 second primers, about 3 second primers to about 4 second primers, about 3 second primers to about 5 second primers, about 3 second primers to about 6 second primers, about 3 second primers to about 7 second primers, about 3 second primers to about 8 second primers, about 3 second primers to about 9 second primers, about 3 second primers to about 10 second primers, about 3 second primers to about 11 second primers, about 3 second primers to about 12 second primers, about 4 second primers to about 5 second primers, about 4 second primers to about 6 second primers, about 4 second primers to about 7 second primers, about 4 second primers to about 8 second primers, about 4 second primers to about 9 second primers, about 4 second primers to about 10 second primers, about 4 second primers to about 11 second primers, about 4 second primers to about 12 second primers, about 5 second primers to about 6 second primers, about 5 second primers to about 7 second primers, about 5 second primers to about 8 second primers, about 5 second primers to about 9 second primers, about 5 second primers to about 10 second primers, about 5 second primers to about 11 second primers, about 5 second primers to about 12 second primers, about 6 second primers to about 7 second primers, about 6 second primers to about 8 second primers, about 6 second primers to about 9 second primers, about 6 second primers to about 10 second primers, about 6 second primers to about 11 second primers, about 6 second primers to about 12 second primers, about 7 second primers to about 8 second primers, about 7 second primers to about 9 second primers, about 7 second primers to about 10 second primers, about 7 second primers to about 11 second primers, about 7 second primers to about 12 second primers, about 8 second primers to about 9 second primers, about 8 second primers to about 10 second primers, about 8 second primers to about 11 second primers, about 8 second primers to about 12 second primers, about 9 second primers to about 10 second primers, about 9 second primers to about 11 second primers, about 9 second primers to about 12 second primers, about 10 second primers to about 11 second primers, about 10 second primers to about 12 second primers, or about 11 second primers to about 12 second primers.

In some cases, the second primer is about 6 nucleotides in length. In some embodiments, the second primer is at least about 3 nucleotides, at least about 4 nucleotides, at least about 5 nucleotides, at least about 6 nucleotides, at least about 7 nucleotides, or at least about 8 nucleotides in length. In some embodiments, the second primer is at most about 8 nucleotides, at most about 7 nucleotides, at most about 6 nucleotides, at most about 5 nucleotides, at most about 4 nucleotides, or at most about 3 nucleotides in length. In some cases, the second primer can be a oligodeoxyribonucleotide. In some cases, the second primer can be a hexamer.

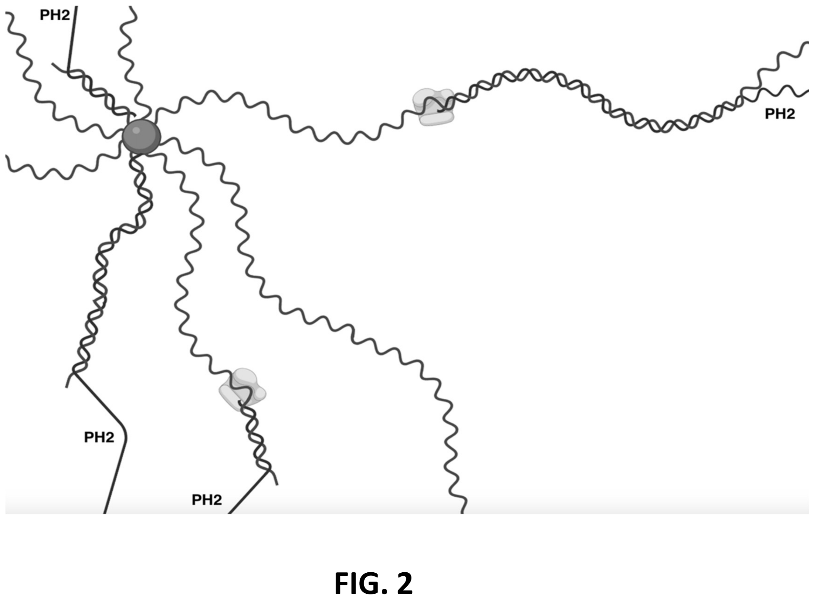

The method can further comprise extending the second primer to generate a second extension strand product. The random nucleotide sequence of the second primer can bind to the first extension strand product in the methods described herein. The second primer can be configured to anneal to the first extension strand product (**FIG. 2**). A nucleic acid amplification can be used to extend the second primer and generate a second extension strand product. In some cases, extending the second primer comprises heating the sample at a constant temperature for a duration of time (*e.g.,* an isothermal incubation). In some cases, the temperature used for synthesizing the second extension strand product is lower than the temperature used for synthesizing the first extension strand product. A low-temperature tolerant enzyme can be used to generate a second extension strand product. In some cases, the low-temperature tolerant enzyme comprises an exo-Klenow polymerase, a Bst 2.0 polymerase and/or a Bst 3.0 polymerase. The low-temperature tolerant enzyme can have strand displacement activity.

In some cases, the constant temperature for the isothermal incubation to generate the second extension strand product can be at least about 20°C, at least about 30°C, at least about 35°C, at least about 40°C, at least about 41°C, at least about 42°C, at least about 43°C, at least about 44°C, at least about 45°C, at least about 46°C, at least about 47°C, at least about 48°C, at least about 49°C, at least about 50°C, at least about 55°C, at least about 60°C, at least about 65°C, at least about 70°C, at least about 75°C, at least about 80°C, at least about 85°C, or at least about 90°C. In some cases, the constant temperature for the isothermal incubation to generate the second extension strand product can be at most about 90°C, at most about 85°C, at most about 80°C, at most about 75°C, at most about 70°C, at most about 65°C, at most about 60°C, at most about 55°C, at most about 50°C, at most about 49°C, at most about 48°C, at most about 47°C, at most about 46°C, at most about 45°C, at most about 44°C, at most about 43°C, at most about 42°C, at most about 41°C, at most about 40°C, at most about 35°C, or at most about 30°C.

In some cases, the constant temperature for the isothermal incubation to generate the second extension strand product can be from about 20°C to about 80°C. In some cases, the constant temperature for the isothermal incubation to generate the second extension strand product can be from about 20°C to about 30°C, about 20°C to about 40°C, about 20°C to about 42°C, about 20°C to about 44°C, about 20°C to about 46°C, about 20°C to about 48°C, about 20°C to about 50°C, about 20°C to about 55°C, about 20°C to about 60°C, about 20°C to about 70°C, about 20°C to about 80°C, about 30°C to about 40°C, about 30°C to about 42°C, about 30°C to about 44°C, about 30°C to about 46°C, about 30°C to about 48°C, about 30°C to about 50°C, about 30°C to about 55°C, about 30°C to about 60°C, about 30°C to about 70°C, about 30°C to about 80°C, about 40°C to about 42°C, about 40°C to about 44°C, about 40°C to about 46°C, about 40°C to about 48°C, about 40°C to about 50°C, about 40°C to about 55°C, about 40°C to about 60°C, about 40°C to about 70°C, about 40°C to about 80°C, about 42°C to about 44°C, about 42°C to about 46°C, about 42°C to about 48°C, about 42°C to about 50°C, about 42°C to about 55°C, about 42°C to about 60°C, about 42°C to about 70°C, about 42°C to about 80°C, about 44°C to about 46°C, about 44°C to about 48°C, about 44°C to about 50°C, about 44°C to about 55°C, about 44°C to about 60°C, about 44°C to about 70°C, about 44°C to about 80°C, about 46°C to about 48°C, about 46°C to about 50°C, about 46°C to about 55°C, about 46°C to about 60°C, about 46°C to about 70°C, about 46°C to about 80°C, about 48°C to about 50°C, about 48°C to about 55°C, about 48°C to about 60°C, about 48°C to about 70°C, about 48°C to about 80°C, about 50°C to about 55°C, about 50°C to about 60°C, about 50°C to about 70°C, about 50°C to about 80°C, about 55°C to about 60°C, about 55°C to about 70°C, about 55°C to about 80°C, about 60°C to about 70°C, about 60°C to about 80°C, or about 70°C to about 80°C.

In some cases, extending the second primer comprises contacting the second primer with deoxynucleoside triphosphates (dNTPs). The dNTPs can be natural or unnatural dNTPs. In some cases, the dNTPs comprise dATP, dCTP, dGTP, dTTP, and/or dUTP. In some cases, the unnatural dNTPs comprise dideoxynucleotide triphosphates (ddNTPs). The ddNTPs can comprise ddGTP, ddATP, ddTTP and ddCTP. The unnatural dNTPs can comprise α-thiol dNTPs (*e.g*., S-dNTPs). S-dNTPS can comprise dATPαS, dCTPαS, dGTPαS, and/or dTTPαS.

The method can further include amplifying the first extension strand product and the second extension strand product with a set of amplification primers to generate a junction-specific amplification product. A first extension strand product and a second extension strand product can include a first primer handle and a second primer handle. In some cases, a first extension strand product includes a first primer handle and a second primer handle. In some cases, a second extension strand product includes a first primer handle and a second primer handle. In some cases, the first extension strand product and a second extension strand product can be contacted with a set of amplification primers and selectively amplify the first extension strand product and a second extension strand product to generate junction-specific amplification product. The amplifying can be a polymerase chain reaction, an isothermal amplification, or another form of nucleic acid amplification.

The set of amplification primers can include index adaptors. An index adaptor can bind to a 5' end of a strand and/or a 3' end of a strand. The index adaptors can include single index adaptors or dual index adaptors. In some cases, the index adaptors include a barcode region, wherein the barcode region can comprise a UMI. Without wishing to be bound by theory, an index adaptor comprising a UMI can help increase variant identification in analysis and identify duplicates from the amplification process. The barcoding region can comprise a barcode and/or a 3' sequence complementary to a hybridization tag of the first primer handle or the second primer handle. The barcode region can also include a microparticle (*e.g*., a bead).

The amplification primers can be from about 20 bases to about 100 bases in length. In some cases, the amplification primers can be from about 20 bases to about 30 bases, about 20 bases to about 40 bases, about 20 bases to about 50 bases, about 20 bases to about 60 bases, about 20 bases to about 70 bases, about 20 bases to about 75 bases, about 20 bases to about 80 bases, about 20 bases to about 85 bases, about 20 bases to about 90 bases, about 20 bases to about 95 bases, about 20 bases to about 100 bases, about 30 bases to about 40 bases, about 30 bases to about 50 bases, about 30 bases to about 60 bases, about 30 bases to about 70 bases, about 30 bases to about 75 bases, about 30 bases to about 80 bases, about 30 bases to about 85 bases, about 30 bases to about 90 bases, about 30 bases to about 95 bases, about 30 bases to about 100 bases, about 40 bases to about 50 bases, about 40 bases to about 60 bases, about 40 bases to about 70 bases, about 40 bases to about 75 bases, about 40 bases to about 80 bases, about 40 bases to about 85 bases, about 40 bases to about 90 bases, about 40 bases to about 95 bases, about 40 bases to about 100 bases, about 50 bases to about 60 bases, about 50 bases to about 70 bases, about 50 bases to about 75 bases, about 50 bases to about 80 bases, about 50 bases to about 85 bases, about 50 bases to about 90 bases, about 50 bases to about 95 bases, about 50 bases to about 100 bases, about 60 bases to about 70 bases, about 60 bases to about 75 bases, about 60 bases to about 80 bases, about 60 bases to about 85 bases, about 60 bases to about 90 bases, about 60 bases to about 95 bases, about 60 bases to about 100 bases, about 70 bases to about 75 bases, about 70 bases to about 80 bases, about 70 bases to about 85 bases, about 70 bases to about 90 bases, about 70 bases to about 95 bases, about 70 bases to about 100 bases, about 75 bases to about 80 bases, about 75 bases to about 85 bases, about 75 bases to about 90 bases, about 75 bases to about 95 bases, about 75 bases to about 100 bases, about 80 bases to about 85 bases, about 80 bases to about 90 bases, about 80 bases to about 95 bases, about 80 bases to about 100 bases, about 85 bases to about 90 bases, about 85 bases to about 95 bases, about 85 bases to about 100 bases, about 90 bases to about 95 bases, about 90 bases to about 100 bases, or about 95 bases to about 100 bases in length.

In some cases, amplifying can be performed *in situ.* In some cases, amplifying can be performed in an intact cell. In some cases, amplifying can be performed on nucleic acids isolated from a cell. The junction-specific amplification product can be barcoded, wherein barcoding comprises analyzing the barcode region of the amplification primers attached to a bead using emulsion PCR. Emulsion PCR can permit amplification of DNA molecules in physically separated picoliter-volume water-in-oil droplets. This procedure can assist in avoiding formation of unproductive chimeras and other artifacts between similar DNA sequences. In some cases, the junction-specific amplification product can be isolated using a capture nucleic acid.

In some cases, amplifying the first extension strand product and the second extension strand product with a set of amplification primers to generate a junction-specific amplification product can comprise performing a library preparation on the junction-specific amplification products. The library preparation can comprise tagmentation or a library PCR using sequencing adaptor primers.

The methods described herein can comprise analyzing the junction-specific amplification product. The junction-specific amplification products can be subjected to sequencing. In some cases, the sequencing can comprise one or more high-throughput sequencing methods. High throughput sequencing methods can comprise Massively Parallel Signature Sequencing (MPSS), polony sequencing, 454 pyrosequencing, Illumina sequencing, SOLiD sequencing, ion semiconductor sequencing, DNA nanoball sequencing, heliscope single molecule sequencing, single molecule real time (SMRT) sequencing, RNAP sequencing, Nanopore DNA sequencing, sequencing by hybridization, or microfluidic Sanger sequencing. In some cases, the junction-specific amplification products can be sequenced using next generation sequencing.

In some cases, analyzing the junction-specific amplification product comprises identifying a junction integration site in the nucleic acid molecule in the genome (e.g., an integration site of the integrated nucleic acid molecule). In some cases, analyzing the junction-specific amplification product comprises identifying a copy number of integration (e.g., sequencing, determination of junctions consisting of at least 30bp of vector flanking DNA, alignment of that flanking sequence to the human genome, identification of unique positions in the human genome as vector insertion sites). Identifying a junction integration site and a copy number of integration from analyzing the junction-specific amplification product can occur concurrently.

### Chimeric Antigen Receptors

Cytotoxic cells expressing chimeric antigen receptors (CARs) can significantly improve the specificity and sensitivity of these cytotoxic cells. A T-cell expressing a CAR (*e.g.,* a CAR-T cell) can utilize the CAR to direct the modified T cell to target tumor cells expressing a cell surface antigen that specifically binds to the CAR, or similarly any target cell expressing a target antigen. CAR-T cells can deliver cytotoxic agents more selectively to the target tumor cells. CAR-T cells can directly recognize a target molecule and are not as restricted by polymorphic presenting elements such as human leukocyte antigens (HLAs). There are three characteristic advantages to the CAR-targeting system: (a) the CAR functions independently of HLA molecules and can target a cell surface antigen present on a tumor cell or invading cell; (b) tumor cells can escape immune system mechanisms through antigen corruption or suppression of presenting molecules, however CAR T-cells can still function and recognize surface antigens; and (c) CARs can be synthesized to recognize a range of macromolecules.

The methods disclosed herein can be used determine a junction integration site of a nucleic acid molecule in a genome and a copy number of integration, wherein the nucleic acid molecule integrated into a genomic DNA molecule includes a CAR. The CAR can include (i) an antigen-binding domain sequence; (ii) a costimulatory domain sequence; (iii) an intracellular signaling domain sequence; and (iv) a 3' genomic junction.

The antigen-binding domain sequence can encode an antigen-binding domain that can bind to a target antigen on a target cell. In some cases, an antigen-binding domain encoded by a CAR molecule of the present disclosure can bind to CD20, CD19, CD22, CD23, CD24, CD30, CD33, CD38, CEA, EGP-2, EGP-40, Eph2, FBP, HMW-MAA, KDR, MUC1, PSCA, ROR1, TAG-72, or VEGR-R2. A costimulatory domain of a CAR can enhance cell proliferation, cell survival, and/or the development of memory cells for the cytotoxic cells that express the CAR. In some cases, a costimulatory domain encoded by a CAR sequence of the present disclosure can comprise CD28, CD137 (4-1BB), CD134 (OX40), Dap10, CD27, CD2, CD7, CD5, ICAM-1, LFA-1(CD1 la/CD18), Lck, TNFR-I, PD-1, TNFR-II, Fas, CD30, CD40, ICOS LIGHT, NKG2C, B7-H3, or combinations thereof. A CAR can have multiple costimulatory domain sequences. A CAR can comprise at least 1, at least 2, at least 3, at least 4, or at least 5 costimulatory domains. Costimulatory domains can be separated by a linker. The CAR can comprise at least a portion of a costimulatory sequence. In some cases, the CAR comprises at least a portion of a CD28 domain sequence or at least a portion of a 4-1BB domain sequence. An intracellular signaling domain of a CAR can help transduce an effector signal and direct the cytotoxic functioning of the T-cell to eliminate or reduce the number of target cells. In some cases, an intracellular signaling domain encoded by a CAR sequence of the present disclosure can comprise CD3γ, CD3ζ, CD3ε, FcR gamma, FcR beta, CD3δ, CD5, CD22, CD79a, CD79b, or CD66d. The intracellular signaling domain can also comprise FcyRIII, FcsRI, cytoplasmic tails of Fc receptors, or an immunoreceptor tyrosine-based activation motif (ITAM). The intracellular signaling domain sequence can comprise a CD3ζ domain sequence.

In some cases, the antigen binding sequence of the CAR can be 5' to the costimulatory domain sequence. The costimulatory sequence can be 5' to the intracellular signaling domain sequence. The CAR can comprise two or more costimulatory sequences. In some cases, at least one costimulatory sequence includes a 4-1BB domain sequence or a portion of a 4-1BB domain sequence.

The first primers described herein can hybridize to a sequence of an integrated nucleic acid molecule encoding a CAR. The first primers can hybridize to (i) an antigen-binding domain sequence; (ii) a costimulatory domain sequence; (iii) an intracellular signaling domain sequence; and (iv) a 3' genomic junction. The first primer can be extended to generate a first extension strand product comprising a sequence comprising (i) an antigen-binding domain sequence; (ii) a costimulatory domain sequence; (iii) an intracellular signaling domain sequence; and (iv) a 3' genomic junction. The first extension strand product can comprise a sequence of the integrated CAR molecule and a sequence of the genomic DNA molecule. A second primer can be configured to hybridize to the 3' end of a intracellular signaling domain sequence. A second primer can be configured to hybridize to a sequence of integrated CAR molecule present on the first extension strand product. The second primer can comprise a UMI, a barcode sequence, and/or a hybridization tag.

Without wishing to be bound by theory, the methods described herein can be used for simultaneous identification of integration site and copy number of an integrated CAR molecule introduced to target cells.

In some aspects, the present disclosure provides methods of quality control on a cell that includes an integrated CAR molecule. The method of quality control can include identifying an junction integration site of the CAR sequence via a sequence of a 3' genomic junction of the junction-specific amplification product. The junction of integration site can be compared to a genomic location oncogene. In some cases, the cell can be rejected when the junction integration site overlaps with an oncogene sequence. In some cases, the cell can be accepted when the junction integration site does not overlap with an oncogene sequence. A method of quality control can further comprise identifying a copy number of integration of the CAR sequence in the genome of the cell by identifying a number of a 3' genomic junction sequence that is unique. The copy number of integration of the CAR can be compared to a threshold copy number. In some cases, a cell can be rejected when the copy number of integration exceeds the threshold. In some cases, a cell can be accepted when the copy number of integration does not exceed the threshold. A threshold vector copy number can be at least about 1, at least about 2, at least about 3, at least about 4, at least about 5, at least about 6, at least about 7, or at least about 8. In some cases, a vector copy number can be more than 10.

In some aspects, the present disclosure provides a non-transitory computer readable storage media comprising instructions for processing sequence reads produced by the methods described herein and using the sequence reads to identify copy number of the CAR or a junction integration site of the CAR. The sequencing methods can comprise one more high-throughput sequencing methods.

### Kits for Analyzing a Cell

In some aspects, the present disclosure provides kits for analyzing a cell that has a sequence of an integrated nucleic acid molecule in its genome. The kits include: (a) a first primer that is configured to hybridize to the sequence of the integrated nucleic acid molecule, wherein the first primer includes a first primer handle having a sequence that does not hybridize to a sequence of the integrated nucleic acid molecule; and (b) a second primer including a second primer handle that is configured to hybridize to an amplification primer, wherein the first primer and the second primer are configured to anneal to a plurality of amplification primers.

The integrated nucleic acid molecule of the kits described herein can include a CAR sequence. The CAR sequence can include an antigen-binding domain sequence; a costimulatory domain sequence; an intracellular signaling domain sequence; and a 3' genomic junction. In some cases, the intracellular signaling domain sequence can be a CD3ζ domain sequence. The first primer of the kit can hybridize to any sequence of the integrated CAR molecule, including but not limited to, the antigen-binding domain sequence; the costimulatory domain sequence; the intracellular signaling domain sequence; and the 3' genomic junction.

In some cases, the first primer of the kit can be a hybrid molecule including a primer handle. The first primer can comprise a first primer handle, comprising a fixed 5' region that cannot bind to the integrated nucleic acid molecule or the genomic DNA molecule. The first primer handle can be biotinylated. Without wishing to be bound by theory, biotinylation of the first primer handle can enrich amplification products. In some cases, the primer is tagged with biotin or 6-carboxyfluorescein (FAM) for visualization on a lateral flow immunoassay strip.

The first primer can also include a barcode sequence and/or a hybridization tag (e.g., hybridization probe). The hybridization tag can include a fragment of DNA or RNA. The hybridization tag can be at least about, at most about, or about 15 nucleotides, 50 nucleotides, 100 nucleotides, 250 nucleotides, 500 nucleotides, 750 nucleotides, 1000 nucleotides, 1500 nucleotides, 2000 nucleotides, or 5000 nucleotides in length. A hybridization tag can be radioactively or fluorescently labeled to detect the presence of nucleotide sequences in analyzed DNA or RNA. Examples of hybridization probes include, but are not limited to, Scorpion^{®} probes, Molecular Beacon probes, TaqMan^{®} probes, Locked Nucleic Acid (LNA^{®}) probes, and Cycling Probe Technology (CPT). In some cases, the first primers described herein can include a unique molecular identifier (UMI).

The first primer of the kit can also include a number of degenerate nucleotides. In some cases, the first primer includes at least about 2 degenerate nucleotides, at least about 3 degenerate nucleotides, at least about 4 degenerate nucleotides, at least about 5 degenerate nucleotides, at least about 6 degenerate nucleotides, at least about 7 degenerate nucleotides, at least about 8 degenerate nucleotides, at least about 9 degenerate nucleotides, at least about 10 degenerate nucleotides, or at least about 12 degenerate nucleotides.

The second primer of the kit can also include a number of degenerate nucleotides. In some cases, the second primer includes at least about 2 degenerate nucleotides, at least about 3 degenerate nucleotides, at least about 4 degenerate nucleotides, at least about 5 degenerate nucleotides, at least about 6 degenerate nucleotides, at least about 7 degenerate nucleotides, at least about 8 degenerate nucleotides, at least about 9 degenerate nucleotides, at least about 10 degenerate nucleotides, or at least about 12 degenerate nucleotides.

In some cases, the kit further includes a reagent for nucleic acid amplification including a thermostable enzyme and/or deoxynucleoside triphosphates (dNTPs). In some cases, the reagent is lyophilized. In some cases, the kit further includes a thermostable enzyme compatible with the primers and methods as described herein. In some cases, the thermostable enzyme of the kit includes strand-displacing activity. In some embodiments, the kit includes a thermostable enzyme selected from the group consisting of a *Bacillus stearothermophilus* polymerase, a large fragment of a *Bacillus stearothermophilus* polymerase, a exo-Klenow polymerase, a Bst 2.0 polymerase, a Bst 3.0 polymerase, an SD DNA polymerase, a phi29 DNA polymerase, a sequencing-grade T7 exo-polymerase, a *Thermus aquaticus* (e.g., Taq-polA), a *Thermotoga maritima* (e.g., Tma-polA), a PfupolB, a Pab-polB, an OmniTaq 2 LA DNA polymerase, and any mutants thereof. The dNTPs of the kit described herein can be natural or unnatural dNTPs. In some cases, the dNTPs comprise dATP, dCTP, dGTP, dTTP, and/or dUTP. In some cases, the unnatural dNTPs comprise di-deoxynucleotide triphosphates (ddNTPs). The ddNTPs can comprise ddGTP, ddATP, ddTTP and ddCTP. The unnatural dNTPs can comprise α-thiol dNTPs (e.g., S-dNTPs). S-dNTPS can include dATPαS, dCTPαS, dGTPαS, and/or dTTPαS.

The kits described herein can further include a capture nucleic acid including a 3' end that includes a CD3ζ domain sequence immobilized on a surface. The surface can a solid support or microparticle. In some cases, the microparticle can be a bead. The capture nucleic acid can further include a barcode sequence 5' to the CD3ζ domain sequence and the barcode sequence can include a UMI. In some cases, the kit further includes a fixative, a permeabilization agent, or a cell lysis agent.

In some cases, the primers and reagents of the kit can be stable at room temperature. In some cases, the primers and reagents of the kit described herein can be stable for at least about 1 day, at least about 2 days, at least about 3 days, at least about 4 days, at least about 5 days, at least about 10 days, at least about 15 days, at least about 30 days, or more.

The kits can be compartmentalized for ease of use and can include one or more containers with reagents. In some embodiments, all of the kit components are packaged together. Alternatively, one or more individual components of the kit can be provided in a separate package from the other kits components.

In some embodiments, the instruction for use includes optimal reaction temperatures for amplification methods, or optimal buffer conditions for the same. The instructions can be in physical (e.g., printed) or electronic form. The instructions can be in print media. As an alternative, the instructions can be accessible by a user on the Internet, such as through a uniform resource locator.

### EXAMPLES

The following examples are provided to further illustrate some embodiments of the present disclosure, but are not intended to limit the scope of the disclosure; it will be understood by their exemplary nature that other procedures, methodologies, or techniques known to those skilled in the art can alternatively be used.

### EXAMPLE 1: Analysis of Amplification Products Following Thermocycling Optimization

In the first stage of this version, a biotinylated forward primer handle (S5) for PCR amplification is installed when 10 linear amplification products are generated using a thermostable strand displacing enzyme (Thermodesulfatator - TIN-001 Pro-Lab Diagnostics) to extend 10 hybrid primers in each of 100 cycles of linear amplification (95-60°C) while limiting the extension time to ~30 sec to bias towards shorter (<1 kb) products. In the sequences below, the underlined nucleotides denote the primer landing sites on the vector template sequences.

### First Round Amp Reaction Mix - TIN

| **Component** | **Final Conc. Or Amount** | **Volume (uL) / 20 uL Rxn** | **Volume (uL) / 50 Rxns** |
|---|---|---|---|
| TIN | 1X | 1 | 50 |
| 10x Buffer 2 | 1X | 2 | 100 |
| MgSO₂ | 5 mM | 2 | 100 |
| dNTP (10mM) | 1 mM | 2 | 100 |
| Forward Oligo Pool (10 uM) | 0.5 uM | 1 | 50 |
| DNA Template | 0, 10 ng | 1 | - |
| dH2O | N/A | 11 | 550 |

### Round 1 Primer Sequences:

| | | |
|---|---|---|
| NS5_3'LTR_Tile-1 | TCGTCGGCAGCGTCGCAGCTGTAGATCTTAGCCAC T | SEQ ID NO: 1 |
| NS5_3'LTR_Tile-2 | TCGTCGGCAGCGTCGGGACTGGAAGGGCTAATTCA | SEQ ID NO: 2 |
| NS5_3'LTR_Tile-3 | TCGTCGGCAGCGTCCCAACGAAGACAAGATCTGCT T | SEQ ID NO: 3 |
| NS5_3'LTR_Tile-4 | TCGTCGGCAGCGTCACTGGGTCTCTCTGGTTAGAC | SEQ ID NO: 4 |
| NS5_3'LTR_Tile-5 | TCGTCGGCAGCGTCAGCCTGGGAGCTCTCTG | SEQ ID NO: 5 |
| NS5_3'LTR_Tile-6 | TCGTCGGCAGCGTCGAACCCACTGCTTAAGCCTCA | SEQ ID NO: 6 |
| NS5_3'LTR_Tile-7 | TCGTCGGCAGCGTCAGCTTGCCTTGAGTGCTTCAA | SEQ ID NO: 7 |
| NS5_3'LTR_Tile-8 | TCGTCGGCAGCGTCTGTGTGCCCGTCTGTTGT | SEQ ID NO: 8 |
| NS5_3'LTR_Tile-9 | TCGTCGGCAGCGTCCTGGTAACTAGAGATCCCTCA GAC | SEQ ID NO: 9 |
| NS5_3'LTR_Tile-10 | TCGTCGGCAGCGTCAGTCAGTGTGGA+AAA+TCTCT +AGCA | SEQ ID NO: 10 |

### (+ indicates LNA modification)

| **3' End of CAR Construct DNA Template** | | **SEQ ID NO.** |
|---|---|---|
| cctttaagaccaatgacttacaag gcagctgtagatcttagccact ttttaaaagaaaaggggggactggaagggctaattcactcccaacgaagacaagatctgctttttgcttgtactgg gtctctctggttagaccagatctgagcctgggagctctctggctaactagggaacccactgcttaagcctcaataaa gcttgccttgagtgcttcaagtagtgtgtgcccgtctgttgtgtgactctggtaactagagatccctcagaccctttta gtcagtgtggaaaatctctagca | | 11 |
| cctttaagaccaatgacttacaaggcagctgtagatcttagccactttttaaaagaaaaggg gggactggaagggctaattca ctcccaacgaagacaagatctgctttttgcttgtactgggtctctctggttagaccagatctgagcctgggagctctct ggctaactagggaacccactgcttaagcctcaataaagcttgccttgagtgcttcaagtagtgtgtgcccgtctgttg tgtgactctggtaactagagatccctcagacccttttagtcagtgtggaaaatctctagca | | 12 |
| cctttaagaccaatgacttacaaggcagctgtagatcttagccactttttaaaagaaaaggggggactggaagggc taattcactc ccaacgaagacaagatctgctt tttgcttgtactgggtctctctggttagaccagatctgagcctgggagctctctggctaactagggaacccactgctta agcctcaataaagcttgccttgagtgcttcaagtagtgtgtgcccgtctgttgtgtgactctggtaactagagatccct cagacccttttagtcagtgtggaaaatctctagca | | 13 |
| cctttaagaccaatgacttacaaggcagctgtagatcttagccactttttaaaagaaaaggggggactggaagggc taattcactcccaacgaagacaagatctgctttttgcttgt actgggtctctctggttagac cagatctgagcctgggagctctctggctaactagggaacccactgcttaagcctcaataaagcttgccttgagtgct tcaagtagtgtgtgcccgtctgttgtgtgactctggtaactagagatccctcagacccttttagtcagtgtggaaaat ctctagca | | 14 |
| cctttaagaccaatgacttacaaggcagctgtagatcttagccactttttaaaagaaaaggggggactggaagggc taattcactcccaacgaagacaagatctgctttttgcttgtactgggtctctctggttagaccagatctg agcctgggagctctctg gctaactagggaacccactgcttaagcctcaataaagcttgccttgagtgcttcaagtagtgtgtgcccgtctgttgt gtgactctggtaactagagatccctcagacccttttagtcagtgtggaaaatctctagca | | 15 |
| cctttaagaccaatgacttacaaggcagctgtagatcttagccactttttaaaagaaaaggggggactggaagggc taattcactcccaacgaagacaagatctgctttttgcttgtactgggtctctctggttagaccagatctgagcctggg agctctctggctaactagg gaacccactgcttaagcctca ataaagcttgccttgagtgcttcaagtagtgtgtgcccgtctgttgtgtgactctggtaactagagatccctcagacc cttttagtcagtgtggaaaatctctagca | | 16 |
| cctttaagaccaatgacttacaaggcagctgtagatcttagccactttttaaaagaaaaggggggactggaagggc taattcactcccaacgaagacaagatctgctttttgcttgtactgggtctctctggttagaccagatctgagcctggg agctctctggctaactagggaacccactgcttaagcctcaataa agcttgccttgagtgcttcaa gtagtgtgtgcccgtctgttgtgtgactctggtaactagagatccctcagacccttttagtcagtgtggaaaatctcta gca | | 17 |
| cctttaagaccaatgacttacaaggcagctgtagatcttagccactttttaaaagaaaaggggggactggaagggc taattcactcccaacgaagacaagatctgctttttgcttgtactgggtctctctggttagaccagatctgagcctggg agctctctggctaactagggaacccactgcttaagcctcaataaagcttgccttgagtgcttcaagtag tgtgtgcccgtctgttgt gtgactctggtaactagagatccctcagacccttttagtcagtgtggaaaatctctagca | | 18 |
| cctttaagaccaatgacttacaaggcagctgtagatcttagccactttttaaaagaaaaggggggactggaagggc taattcactcccaacgaagacaagatctgctttttgcttgtactgggtctctctggttagaccagatctgagcctggg agctctctggctaactagggaacccactgcttaagcctcaataaagcttgccttgagtgcttcaagtagtgtgtgccc gtctgttgtgtgact ctggtaactagagatccctcagac ccttttagtcagtgtggaaaatctctagca | | 19 |
| cctttaagaccaatgacttacaaggcagctgtagatcttagccactttttaaaagaaaaggggggactggaagggc taattcactcccaacgaagacaagatctgctttttgcttgtactgggtctctctggttagaccagatctgagcctggg agctctctggctaactagggaacccactgcttaagcctcaataaagcttgccttgagtgcttcaagtagtgtgtgccc gtctgttgtgtgactctggtaactagagatccctcagacccttttagtcagtgtggaaaatctctagca | | 20 |
| | | |

| **Stage 1 Products** | | **SEQ ID NO.** |
|---|---|---|
| NS5_ 3'LT R_Til e-1 | TCGTCGGCAGCGTC- GCAGCTGTAGATCTTAGCCACTttttaaaagaaaaggggggactggaagggctaattcactc ccaacgaagacaagatctgctttttgcttgtactgggtctctctggttagaccagatctgagcctgggag ctctctggctaactagggaacccactgcttaagcctcaataaagcttgccttgagtgcttcaagtagtgt gtgcccgtctgttgtgtgactctggtaactagagatccctcagacccttttagtcagtgtggaaaatctct agca...NNNNNNNNN | 21 |
| NS5_ 3'LT R_Til e-2 | TCGTCGGCAGCGTC- GGGACTGGAAGGGCTAATTCActcccaacgaagacaagatctgctttttgcttgtactgggtc tctctggttagaccagatctgagcctgggagctctctggctaactagggaacccactgcttaagcctcaa taaagcttgccttgagtgcttcaagtagtgtgtgcccgtctgttgtgtgactctggtaactagagatccct cagacccttttagtcagtgtggaaaatctctagca...NNNNNNNNN | 22 |
| NS5_ 3'LT R_Til e-3 | TCGTCGGCAGCGTC- CCAACGAAGACAAGATCTGCTTtttgcttgtactgggtctctctggttagaccagatctgagcc tgggagctctctggctaactagggaacccactgcttaagcctcaataaagcttgccttgagtgcttcaag tagtgtgtgcccgtctgttgtgtgactctggtaactagagatccctcagacccttttagtcagtgtggaaa atctctagca...NNNNNNNNN | 23 |
| NS5_ 3'LT R_Til e-4 | TCGTCGGCAGCGTC- ACTGGGTCTCTCTGGTTAGACactgggtctctctggttagaccagatctgagcctgggagctct ctggctaactagggaacccactgcttaagcctcaataaagcttgccttgagtgcttcaagtagtgtgtgc ccgtctgttgtgtgactctggtaactagagatccctcagacccttttagtcagtgtggaaaatctctagca ...NNNNNNNNN | 24 |
| NS5_ 3'LT R_Til e-5 | TCGTCGGCAGCGTC- AGCCTGGGAGCTCTCTGagcctgggagctctctggctaactagggaacccactgcttaagcctc aataaagcttgccttgagtgcttcaagtagtgtgtgcccgtctgttgtgtgactctggtaactagagatcc ctcagacccttttagtcagtgtggaaaatctctagca...NNNNNNNNN | 25 |
| NS5_ 3'LT R_Til e-6 | TCGTCGGCAGCGTC- GAACCCACTGCTTAAGCCTCAataaagcttgccttgagtgcttcaagtagtgtgtgcccgtctgt tgtgtgactctggtaactagagatccctcagacccttttagtcagtgtggaaaatctctagca...NNNN NNNNN | 26 |
| NS5_ 3'LT R_Til e-7 | TCGTCGGCAGCGTC- AGCTTGCCTTGAGTGCTTCAAgtagtgtgtgcccgtctgttgtgtgactctggtaactagagat ccctcagacccttttagtcagtgtggaaaatctctagca...NNNNNNNNN | 27 |
| NS5_ 3'LT R_Til e-8 | TCGTCGGCAGCGTC- TGTGTGCCCGTCTGTTGTgtgactctggtaactagagatccctcagacccttttagtcagtgtgg aaaatctctagca...NNNNNNNNN | 28 |
| NS5_ 3'LT R_Til e-9 | TCGTCGGCAGCGTC- CTGGTAACTAGAGATCCCTCAGACccttttagtcagtgtggaaaatctctagca...NNNN NNNNN | 29 |
| NS5_ 3'LT RTil e-10 | TCGTCGGCAGCGTC-AGTCAGTGTGGAAAATCTCTAGCA...NNNNNNNNN | 30 |

| **Thermal Cycling (v4)** | | | | | |
|---|---|---|---|---|---|
| **Step** | **Temp** | | **Time** | | |
| **Initial Denaturation** | 95°C | | 3 min | | |
| **Denaturation** | 95°C | | 10 sec | | |
| **Anneal/Extend** | 60°C | | 1 sec | | |
| **Hold** | 4°C | | ∞ | | |
| **Cycles (Denat/Extend)** | 100 | | | | |
| **Ramp rate** | 3°C/sec | | | | |
| **RND-1** | **Volume Recovered (uL)** | **Volume M270 Strep Beads Per Capture Reaction (uL)** | **Volume M270 Strep Beads Post Clean-Up (uL)** | **Volume M270 Strep Beads Post Clean-Up and Strip Per PCR (uL)** | **Total Dilution Factor into PCR** |
| | 50 | 100 | 200 | 25 | 8 |

Junction-specific amplification products were analyzed following optimization of the first amplification round, in which first extension strand products were generated from the first primer set, captured and washed on M270 streptavidin beads, and then used as tempates for a subsequent quality control amplication using qPCR.

### QC Assay - Rnd1_QCv1 (PrimeTime)

| | | |
|---|---|---|
| **FWD:** | CCCACTGCTTAAGCCTCAATA | SEQ ID NO: 31 |
| **REV:** | GGGTCTGAGGGATCTCTAGTT | SEQ ID NO: 32 |
| **PRB:** | /56-FAM/AAGTAGTGT/ZEN/GTGCCCGTCTGTTGT/3IABkFQ/ | SEQ ID NO: 33-34 |

Amplification products were shorter in length **FIG. 3B****.** following reaction optimization, allowing for more efficient subsequent sequencing library preparation steps.

### EXAMPLE 2: Results of Use of Unnatural Nucleotides in Amplification Reactions

In a first experiment, either 100% or 1% of the cytosine nucleotides were replaced with the dideoxy analogue, dideoxycytosine (ddCTP), that cannot be extended once incorporated. Natural dATP, dTTP, and dGTP were present and equimolar to total dCTP/ddCTP.

### 20230313 VCN/IS Assay_v2: RND-1 Nucleotide Pool Variations

Test various unnatural nucleotides and natural nucleotide pool variations to determine their effect on shortening RND-1 product length.

Maintain the same RND-1 reaction conditions except for the composition or concentration of the nucleotide pool.

Make a dNTP stock in which dATP, dTTP, and dGTP are at 10mM, then add natural dCTP and unnatural CTP up to 10mM.

| | **Dilution** | | | |
|---|---|---|---|---|
| **Nucleotide** | **1:1** | **1:10** | **1:100** | **1:1000** |
| 1) ddCTP | + | + | + | + |
| 2) ThioCTP | + | + | + | + |
| 3) dCTP | - | + | + | + |
| 4) dNTP | + | + | + | + |

| | **Final Nucleotide Pool Concentration (mM)** | | | | |
|---|---|---|---|---|---|
| **Nucleotide** | **1:1** | **1:10** | **1:100** | **1:1000** | |
| 1) ddCTP | 1 | 0.1 | 0.01 | 0.001 | Total Concentration 1mM |
| 2) ThioCTP | 1 | 0.1 | 0.01 | 0.001 | |
| 3) dCTP | - | 0.1 | 0.01 | 0.001 | |
| 4) dNTP | 1 | 0.1 | 0.01 | 0.001 | Total Concentration Varies |

| **First Round Amp Reaction Mix - TIN** | | | |
|---|---|---|---|
| **Component** | **Final Conc. Or Amount** | **Volume (uL) / 20 uL Rxn** | **Volume (uL) / 50 Rxns** |
| TIN | 1X | 1 | 50 |
| 10x Buffer 2 | 1X | 2 | 100 |
| MgSO2 | 5 mM | 2 | 100 |
| dNTP (10mM) | 1mM | 2 | 100 |
| Forward Oligo Pool (10 uM) | 0.5 uM | 1 | 50 |
| DNA Template | 0, 20 ng | 10 | N/A |
| dH2O | N/A | 2 | 100 |

| **Thermal Cycling (v4)** | | |
|---|---|---|
| **Step** | **Temp** | **Time** |
| **Initial Denaturation** | 95°C | 3 min |
| **Denaturation** | 95°C | 10 sec |
| **Anneal/Extend** | 60°C | 1 sec |
| **Hold** | 4°C | ∞ |
| **Cycles (Denat/Extend)** | 100 | |
| **Ramp rate** | 3°C/sec | |

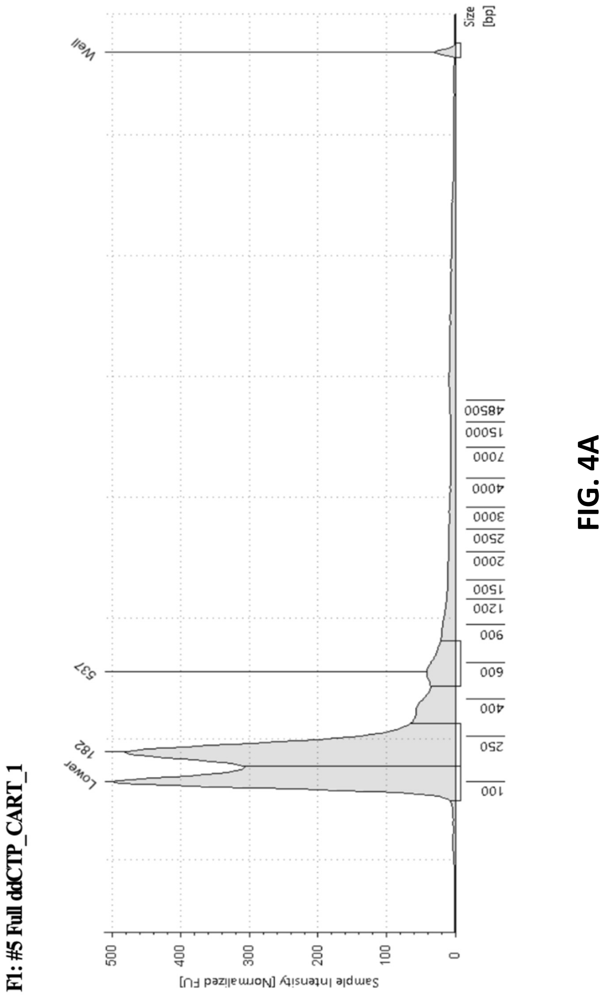

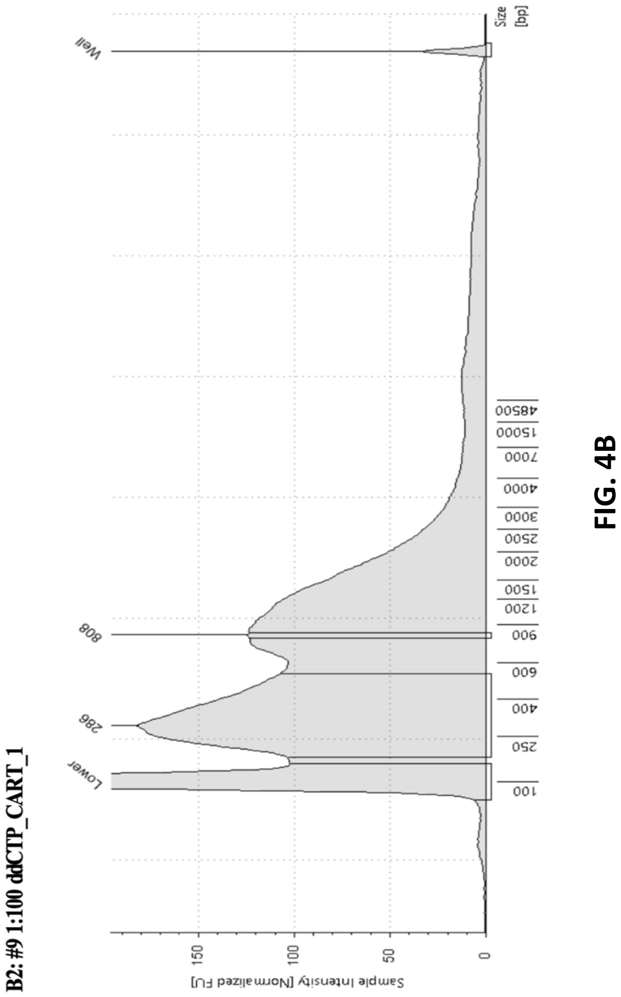

Results in **FIGs. 4A-4B** show resulting amplification products were of shorter length than those from reactions with natural dNTPs. In the reaction with 100% ddCTP **(****FIG. 4A****),** the amplification products were the shortest length demonstrating that introduction of unnatural dNTPs can control the length of resulting amplicons. Importantly, the addition of unnatural nucleotides further restricts the extremely long extension products resulting from the optimization RND-1 amplification reaction into a population suitable for futher library preparation.

These results indicate that amplicons need only include the viral/human junction site and enough human genomic sequence to be identified as a unique location (>30 bp as indicated by the UCSC genome browser). Any extra sequence (conservatively past ~100 bp) is unnecessary and hampers NGS library construction and quality and sequencing efficiency.

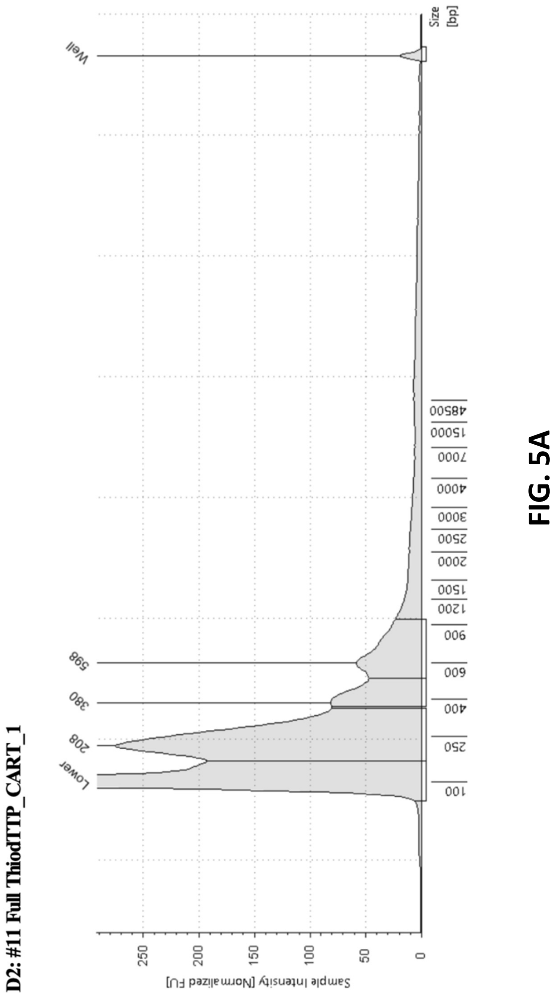

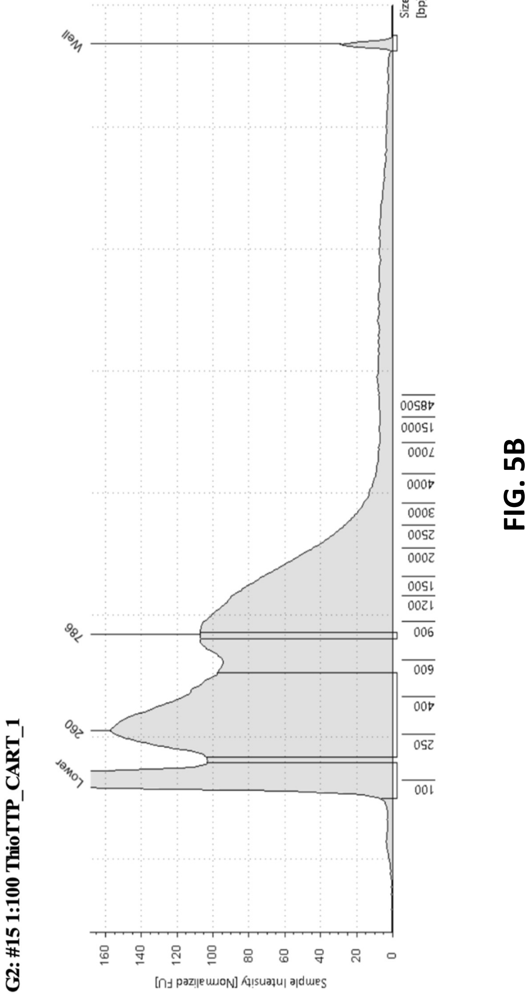

In a second experiment, either 100% or 1% of the thymidine nucleotides were replaced with the α-thio analogue (dTTPαS) that slows the rate of incorporation by DNA polymerase. Natural dATP, dCTP, & dGTP were present and equimolar to total dTTP/dTTPαS. Results in **FIGs. 5A-5B** show resulting amplification products were of similar or shorter length than those from reactions with natural dNTPs. Amplification products from the reaction with 100% dTTPαS **(****FIG. 5A****)** showed shorter lengths than those from the reaction with 1% dTTPαS **(****FIG. 5B****),** demonstrating introduction of unnatural dNTPs can control the length of resulting amplicons.

### EXAMPLE 3: Results of Quantitative PCR of Reaction Products

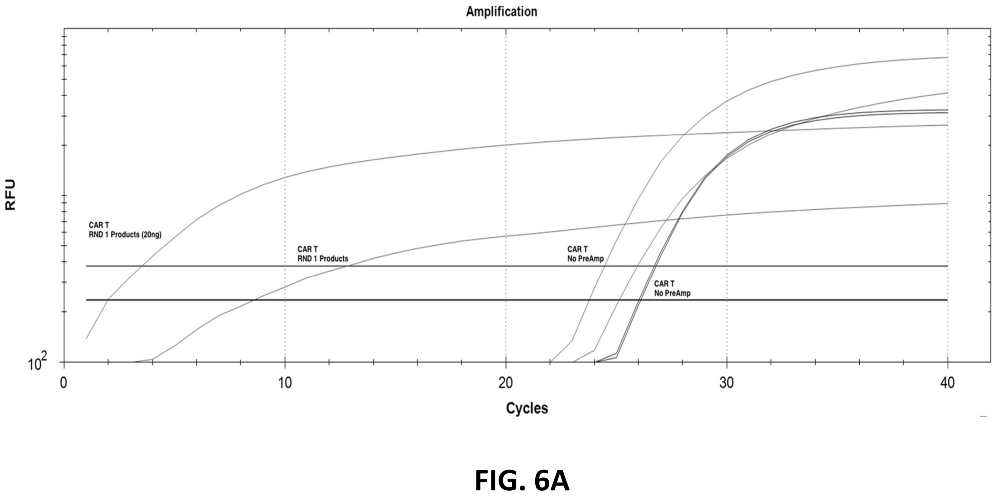

Reaction products from the first round of amplification of template genomic DNA extracted from a CAR T cell sample were checked by quantitative PCR using either custom primer/probe set specific to the 3' LTR region of the viral construct (circles) or using published VCN assay primer/probe sets against the viral PSI region (triangles) or the single copy human ALB gene (squares). Undiluted first round reaction product crossed the threshold >20 cycles before the published assay when equal amounts of starting template were used, indicating >2^20 fold enrichment. **(****FIG. 6A****)**

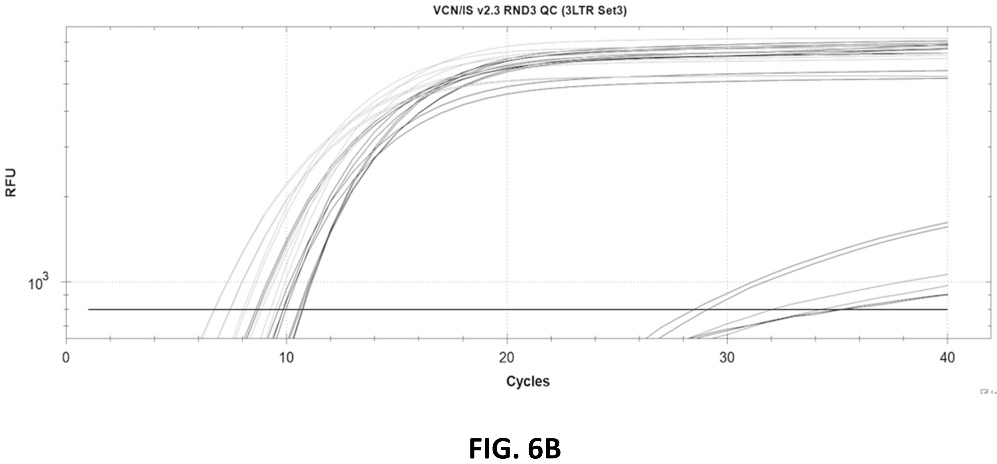

To demonstrate assay specificity, control samples using either no template (NTC - crosses), 3000 copies of artificial dsDNA containing an artificial viral/human junction (gBLK - squares), or 10 ng of human gDNA extracted from peripheral blood monocytes (PBMC - triangles) were amplified alongside 10 ng of CAR-T gDNA test samples (CAR-T - circles) in a first round amplification. NTC and PBMC reactions were negative, while gBLK and CAR-T reactions were comparably positive and well above background. **(****FIG. 6B****)**

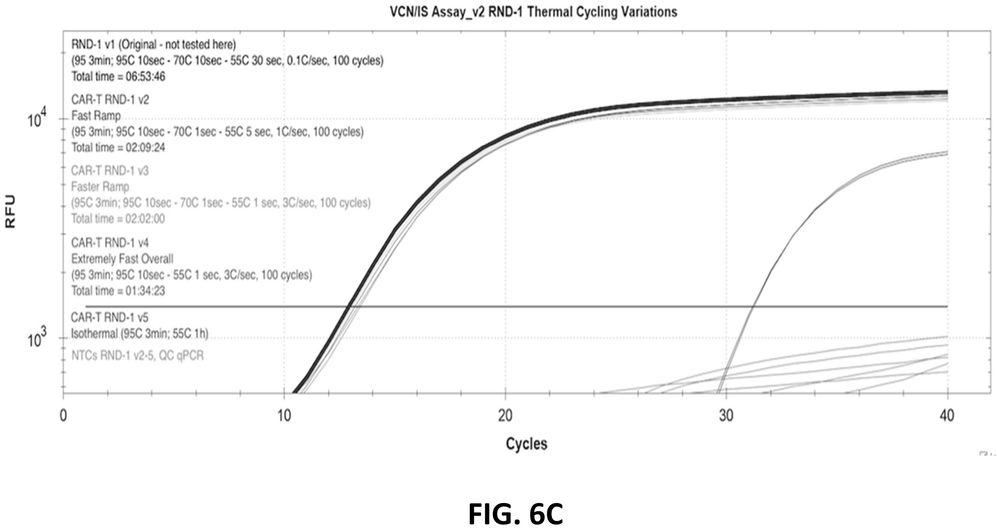

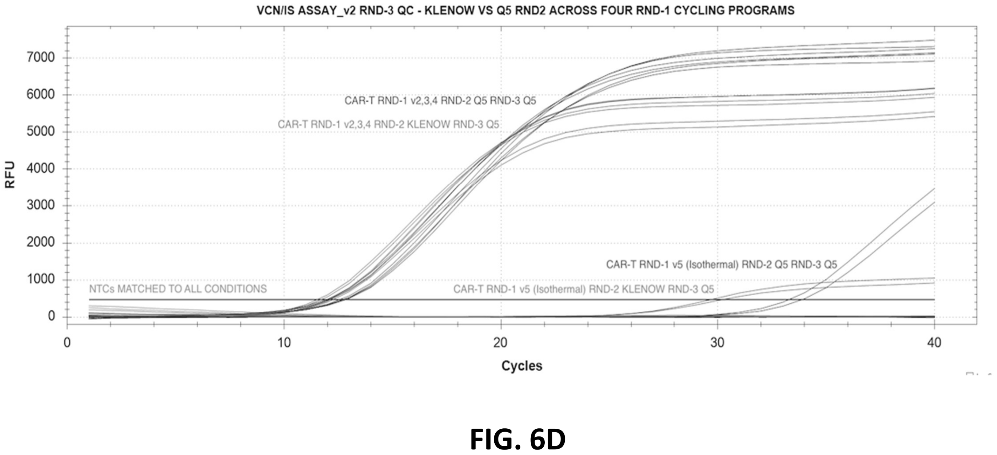

To reduce the overall reaction time from ~7h to ~1h and shortening the amplification product lengths, various first-round amplification thermal cycling programs were tested and checked by quantitative PCR. Reaction templates were CAR T gDNA (circles), PBMC gDNA (triangles), NTC (crosses) **(****FIG. 6C****).** These first-round reaction products were then taken through second round amplification using either klenow (circles) or Q5 (squares) polymerase to install a second amplification handle, and then round three amplification to complete the NGS adaptor installation library and finally checked by quantitative PCR. Negative controls included PBMC gDNA (triangles) and NTC (crosses) reactions. **(****FIG. 6D****).** Modifying the first-round amplification thermal cycling conditions did not impact sensitivity, but resulted in an optimized reaction that reduced time by ~5.5h and product lengths by ~5-10 fold.

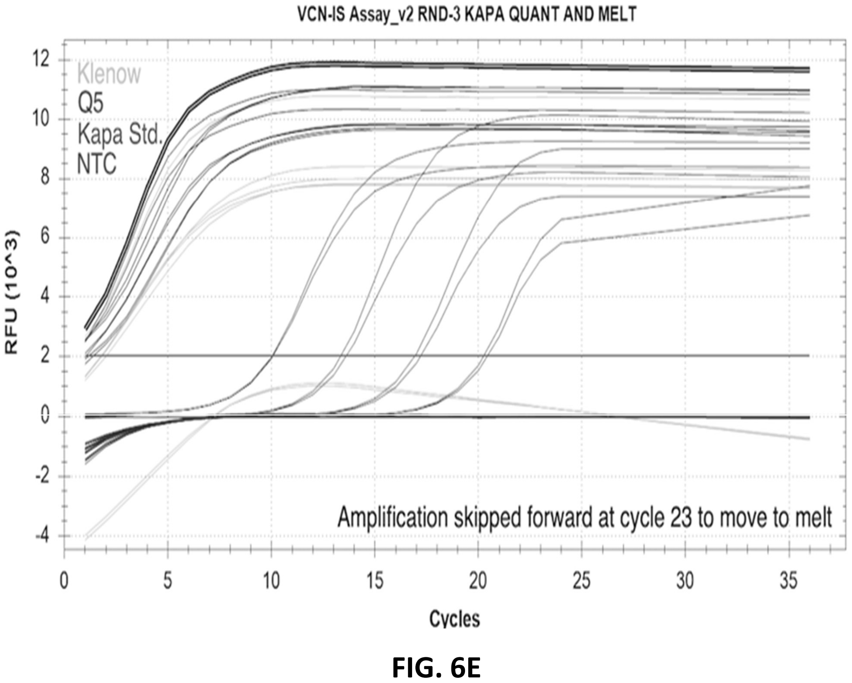

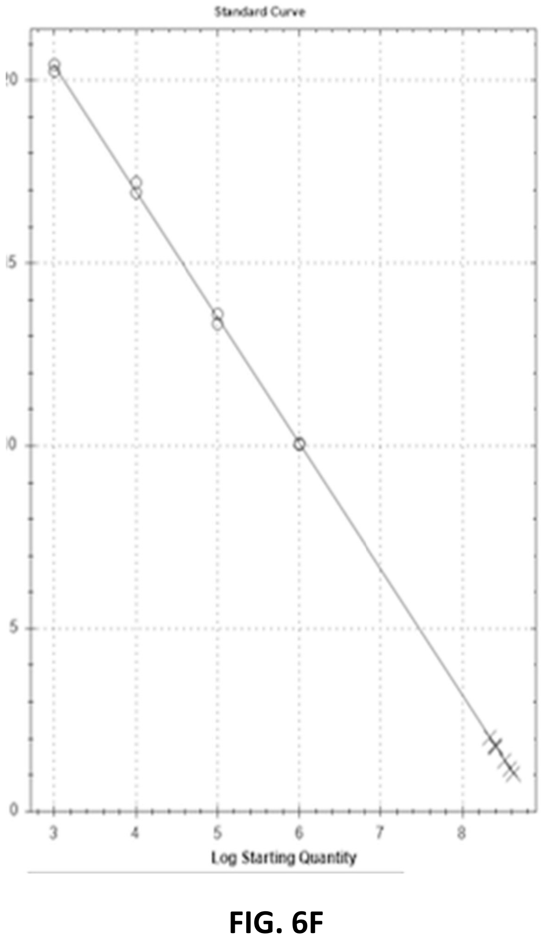

Third-round amplification products were functionally tested and quantified using an Illumina NGS library specific PCR from Kapa Biosystems. All reactions yield a high concentration of NGS competent products. **(****FIG. 6E****,** **FIG. 6F****)**

### OTHER EMBODIMENTS

It is to be understood that the foregoing description is intended to illustrate and not limit the scope of the present disclosure, which is defined by the scope of the appended claims. Other aspects, advantages, and modifications are within the scope of the following claims.

## Claims

1. A method for determining a junction integration site of a nucleic acid molecule in a genome and a copy number of integration, the method comprising:
(a) providing a sample of a genomic DNA molecule from a cell, wherein the genomic DNA molecule comprises an integrated nucleic acid molecule;
(b) contacting a sequence of the integrated nucleic acid molecule with a first primer,
wherein the first primer is configured to hybridize to the integrated nucleic acid molecule, and
wherein the first primer comprises a first primer handle comprising a sequence that does not hybridize to the integrated nucleic acid molecule;
(c) extending the first primer to generate a first extension strand product comprising a sequence of the integrated nucleic acid molecule and a sequence of the genomic DNA molecule;
(d) contacting the first extension strand product with a second primer,
wherein the second primer is a random primer configured to anneal to the first extension strand product, and
wherein the second primer comprises a second primer handle configured to hybridize to an amplification primer;
(e) extending the second primer to generate a second extension strand product;
(f) amplifying the first extension strand product and the second extension strand product with a set of amplification primers to generate a junction-specific amplification product, wherein the set of amplification primers comprises index adaptors; and
(g) identifying the junction integration site in the nucleic acid molecule in the genome, and identifying a copy number of integration,
wherein identifying the junction of integration and identifying a copy number of integration occur concurrently, and wherein identifying the junction of integration and identifying a copy number of integration occur at a single cell resolution.

2. The method of claim 1, wherein the cell is a T-cell, a natural killer cell, a precursor of a T-cell, or a precursor of a natural killer cell.

3. The method of claim 1 or claim 2, wherein the first primer handle:
i) is biotinylated,
ii) comprises a barcode sequence,
iii) comprises a hybridization tag,
iv) comprises a unique molecular identifier (UMI),
v) comprises at least six degenerate nucleotides,
vi) is at least 20 nucleotides in length,
vii) binds to a 3' LTR region of the integrated nucleic acid molecule, or
viii) a combination thereof.

4. The method of any one of claims 1-3, wherein the method comprises use of a plurality of first primers.

5. The method of any one of claims 1-4, wherein extending the first primer in (c) further comprises contacting the sample with a thermostable enzyme and deoxynucleoside triphosphates (dNTPs), wherein the thermostable enzyme comprises an enzyme with strand displacement activity.

6. The method of any one of claims 1-5, wherein the first extension strand product is bound to a bead.

7. The method of any one of claims 1-6, wherein the second primer:
i) comprises a unique molecular identifier (UMI),
ii) comprises a barcode sequence
iii) comprises a hybridization tag,
iv) comprises a random sequence,
v) comprises at least five degenerate nucleotides,
vi) comprises a fixed 5' region,
vii) ends with at least five to nine random nucleotides,
viii) is at least 20 nucleotides in length, or
ix) a combination thereof.

8. The method of any one of claims 1-7, wherein extending the second primer in (e) further comprises contacting the sample with an exo-Klenow polymerase.

9. The method of any one of claims 1-8, wherein the integrated nucleic acid molecule comprises a chimeric antigen receptor (CAR) sequence comprising:
(i) an antigen-binding domain sequence;
(ii) a costimulatory domain sequence;
(iii) an intracellular signaling domain sequence; and
(iv) a 3' genomic junction.

10. The method of claim 9, wherein the first primer is configured to hybridize to the antigen-binding domain sequence, the costimulatory domain sequence, the intracellular signaling domain sequence, the 3' genomic junction, or a combination thereof.

11. The method of claim 10, wherein the second primer comprises a 3' end configured to hybridize to a 3' end of the intracellular signaling domain sequence.

12. The method of any one of claims 1-11, wherein the amplifying is performed *in situ* or in an intact cell.

13. The method of any one of claims 1-12, wherein the genomic DNA molecule comprises a chimeric antigen receptor (CAR) sequence, and wherein the method further comprises:
identifying a junction integration site of the CAR sequence via a sequence of a 3' genomic junction of the junction-specific amplification product;
comparing the junction integration site to a genomic location oncogene; and
rejecting the cell when the junction integration site overlaps with an oncogene sequence.

14. The method of claim 13, further comprising:
identifying a copy number of integration of the CAR sequence in the genome of the cell by identifying a number of unique 3' genomic junction sequences;
comparing the copy number of integration to a threshold and;
rejecting the cell when the copy number of integration exceeds the threshold.

15. A kit for performing the method of any one of claims 1-14, the kit comprising:
the first primer; and
the second primer;
wherein the first primer and the second primer are configured to anneal to a plurality of amplification primers.
